# EUROPEAN PATENT APPLICATION

(11) **EP 2 511 706 A1**
(43) Date of publication of application: **17.10.2012**
(21) Application number: 09851877.2
(22) Date of filing: 02.12.2009
(51) Int. Cl.: G01N 33/68, G01N 33/15, C12N 5/10

(54) **MICRORNA REGULATING THE INSULIN SIGNALING PATHWAY, AND METHOD FOR SCREENING MATERIAL FOR CONTROLLING THE ACTION OF A TARGET THEREOF**

(71) Applicant: SNU R & DB Foundation, Gwanak-gu, Seoul 151-742 (KR)
(72) Inventor: KIM, Vic Narry, Seoul 135-836 (KR); LEE, Jung Hyun, Seoul 135-785 (KR); HYUN, Seogang, Seoul 156-761 (KR); JIN, Hua, Seoul 151-813 (KR)
(74) Representative: Winkler, Andreas Fritz Ernst
(86) International application number: PCT/KR2009/007161
(87) International publication number: WO 2011/068260

(57) **Abstract**

The present invention relates to a miRNA regulating the insulin signaling pathway, and to a method for screening a material for controlling the action of a target gene thereof, and particularly, to a method for screening a material for controlling the action of USH or FOG2, a target gene of miR-8 or miR-200 miRNA for promoting cell growth. The present inventors discovered miR-8, a conserved miRNA for regulating the body of a fruit fly by targeting u-shaped material (USH) in the fat cells of Drosophila. It was also confirmed that a target gene of miR-200, a human homologous gene of Drosophila miR-8 miRNA, is FOG2. It was found that Drosophila miR-8 and USH are also conserved in mammals, and FOG2, a human homologous gene of USH, directly binds to a regulating subunit of PI3K and functions. It was confirmed that when the expression of miR-200 is inhibited or FOG2 is expressed in a human cancer cell line, the activity of P13K, which promotes cell growth, is decreased. Therefore, miR-200 and FOG2 may be useful in screening regulators of insulin signaling pathways.

## Description

### [Technical Field]

The present disclosure relates to a method for screening material for controlling the insulin signaling pathway.

### [Background Art]

Animal body size is a biological parameter subject to considerable stabilizing selection; animals of abnormal size are strongly selected against as less fit for survival. Thus, the way in which body size is determined and regulated is a fundamental biological question. Recent studies using insect model systems have begun to provide some clues by showing that insulin signaling plays an important part in modulating body growth (Ikeya et al., 2002, Rulifson et al., 2002). The binding of insulin (insulin-like peptides in *Drosophila*) to its receptor (InR) triggers a phosphorylation cascade involving the insulin receptor substrate (IRS; chico in *Drosophilia*), phosphoinositide-3 kinase (PI3K), and Akt/PKB (Edgar, 2006). An active PI3K complex consists of a catalytic subunit (p110; dp110 in *Drosophila*) and a regulatory subunit (p85a; dp60 in Drosophila). Phosphorylated Akt (p-Akt) phosphorylates many proteins including forkhead box O transcription factor (FOXO) .which are involved in cell death, cell proliferation, metabolism, and life span control (Arden, 2008). Once activated, the kinase cascade enhances cell growth and proliferation.

Organismal growth is achieved not only by cell-autonomous regulation but also by non-cell-autonomous control through circulating growth hormones (Baker et al., 1993; Edgar, 2006). Recent studies in insects indicate that several endocrine organs, such as the prothoracic gland and fat body, govern organismal growth by coordinating developmental and nutritional conditions (Caldwell et al., 2005; Colombani et al., 2005; Colombani et al., 2003; Mirth et al., 2005). However, detailed mechanisms of how body size is determined and modulated remain largely unknown.

microRNAs (miRNAs) are noncoding RNAs of -22 nt that act as posttranscriptional repressors by base-pairing to the 30 untranslated region (UTR) of their cognate mRNAs (Bartel, 2009). The physiological functions of individual miRNAs remain largely unknown. Studies of miRNA function rely heavily on computational algorithms that predict target genes (John et al., 2004; Kim et al., 2006; Kiriakidou et al., 2004; Krek et al., 2005; Lewis et al., 2005; Stark et al., 2003). In spite of their utility, however, these target prediction programs generate many false-positive results, because regulation in vivo depends on target message availability and complementary sequence accessibility. To overcome the difficulties in identifying real targets, various experimental approaches have been developed, including micro-arrays, proteomic analyses, and biochemical purification of the miRNA-mRNA complex (Bartel, 2009). Genetic approaches using model organisms can also be useful tools for studying the biological roles of miRNAs at both the organismal and molecular levels (Smibert and Lai, 2008). Despite these advances, however, it is still a daunting task to understand the biological function of a given miRNA and to identify its physiologically relevant targets.

Here, the inventors of the present disclosure found using Drosophila as a model system that conserved miRNA miR-8 positively regulates body size by targeting a fly gene called u-shaped (ush) in fat body cells. The inventors further discover that this function of miR-8 and USH is conserved in mammals and that the human homolog of USH, FOG2, acts by directly binding to the regulatory subunit of PI3K. Moreover, the inventors identify the miR-200, a human homologue gene of Drosophila miR-8 miRNA, is FOG2. The present inventors find that either the inhibition of miR-200 expression or the induction of FOG2 expression in human carcinoma cells contributes to decrease in PI3K activity (capable of promoting cell proliferation) to reduce cell proliferation, indicating that miR-200 and FOG2 are usefully adopted for screening systems to Identify a substance capable of controlling insulin signal pathway.

Throughout the specification, a number of publications and patent documents are referred to and cited. The disclosure of the cited publications and patent documents is incorporated herein by reference in its entirety to more clearly describe the state of the related art and the present disclosure.

### [Disclosure]

### [Technical Problem]

An object of the present invention is to provide a method of screening an insulin signaling regulator, the method including the steps of:
1) treating cell lines expressing miR-200 family miRNAs or miR-8 miRNA with a testing compound;
2) measuring an expression level or an activity of FOG2 or USH protein in cells treated with the testing compound in the step 1); and
3) identifying the testing compound by which the expression or the activity of the FOG2 or USH protein in the cells in the step 1) has been changed as compared to a control.

Another object of the present invention is to provide a method of screening an insulin signaling regulator, the method including the steps of:
1) treating cell lines expressing FOG2 or USH with a testing compound;
2) measuring an expression level or an activity of FOG2 or USH protein in cells in the step 1); and
3) identifying the testing compound by which the expression or the activity of the FOG2 or USH protein in the cells in the step 1) has been changed as compared to a control.

A further object of the present invention is to provide a method of screening an insulin signaling regulator, the method including the steps of:
1) bringing FOG2 protein into contact with p85α protein in the presence of a testing compound;
2) measuring a degree of binding of the FOG2 protein to the p85α protein;
   and
3) identifying the testing compound by which the degree of binding of the FOG2 protein to the p85α protein in vitro in the step 2) has been changed as compared to a control not treated with the testing compound.

A still further object of the present invention is to provide a method of screening an insulin signaling regulator, the method including the steps of:
1) bringing USH protein into contact with dp60 protein in the presence of a testing compound;
2) measuring a degree of binding of the USH protein to the dp60 protein; and
3) identifying the testing compound by which the degree of binding of the USH protein to the dp60 (Drosophila p60) protein in vitro in the step 2) has been changed as compared to a control not treated with the testing compound.

A yet further object of the present invention is to provide a method of screening an insulin signaling regulator, the method including the steps of:
1) treating cell lines expressing FOG2 protein with a testing compound;
2) measuring a degree of binding of FOG2 protein to p85α protein in cells treated with the testing compound in the step 1); and
3) identifying the testing compound by which the degree of binding of the FOG2 protein to the p85α protein in the cells in the step 1) has been changed as compared to a control not treated with the testing compound.

A still yet further object of the present invention is to provide a method of screening an insulin signaling regulator, the method including the steps of:
1) treating cell lines expressing USH protein with a testing compound;
2) measuring a degree of binding of USH protein to dp60 protein in cells treated with the testing compound in the step 1); and
3) identifying the testing compound by which the degree of binding of the USH protein to the dp60 protein in the cells in the step 1) has been changed as compared to a control not treated with the testing compound.

Another still yet further object of the present invention is to provide an insulin signaling regulating composition including a material changing an expression or an activity of FOG2 or USH protein, as an active ingredient.

Another still yet further object of the present invention is to provide a use of a material changing an expression or an activity of FOG2 or USH protein, in preparation of an insulin signaling regulating composition.

### [Technical Solution]

In accordance with an aspect of the present invention, there is provided a method of screening an insulin signaling regulator, the method including the steps of:
1) treating cell lines expressing miR-200 family miRNAs or miR-8 miRNA with a testing compound;
2) measuring an expression level or an activity of FOG2 or USH protein in cells treated with the testing compound in the step 1); and
3) identifying the testing compound by which the expression or the activity of the FOG2 or USH protein in the cells in the step 1) has been changed as compared to a control.

In accordance with another aspect of the present invention, there is provided a method of screening an insulin signaling regulator, the method including the steps of:
1) treating cell lines expressing FOG2 or USH with a testing compound;
2) measuring an expression level or an activity of FOG2 or USH protein in cells in the step 1); and
3) identifying the testing compound by which the expression or the activity of the FOG2 or USH protein in the cells in the step 1) has been changed as compared to a control.

In accordance with a further object of the present invention, there is provided a method of screening an insulin signaling regulator, the method including the steps of:
1) bringing FOG2 protein into contact with p85α protein in the presence of a testing compound;
2) measuring a degree of binding of the FOG2 protein to the p85α protein; and
3) identifying the testing compound by which the degree of binding of the FOG2 protein to the p85α protein in vitro in the step 2) has been changed as compared to a control not treated with the testing compound.

In accordance with a still further object of the present invention, there is provided a method of screening an insulin signaling regulator, the method including the steps of:
1) bringing USH protein into contact with dp60 protein in the presence of a testing compound;
2) measuring a degree of binding of the USH protein to the dp60 protein; and
3) identifying the testing compound by which the degree of binding of the

USH protein to the dp60 (Drosophila p60) protein in vitro in the step 2) has been changed as compared to a control not treated with the testing compound.

In accordance with a yet further object of the present invention, there is provided a method of screening an insulin signaling regulator, the method inducing the steps of:
1) treating cell lines expressing FOG2 protein with a testing compound;
2) measuring a degree of binding of FOG2 protein to p85α protein in cells treated with the testing compound in the step 1); and
3) identifying the testing compound by which the degree of binding of the FOG2 protein to the p85α protein in the cells in the step 1) has been changed as compared to a control not treated with the testing compound.

In accordance with a still yet further object of the present invention, there is provided a method of screening an insulin signaling regulator, the method including the steps of:
1) treating cell lines expressing USH protein with a testing compound;
2) measuring a degree of binding of USH protein to dp60 protein in cells treated with the testing compound in the step 1); and
3) identifying the testing compound by which the degree of binding of the USH protein to the dp60 protein in the cells in the step 1) has been changed as compared to a control not treated with the testing compound.

In accordance with another still yet further object of the present invention, there is provided an insulin signaling regulating composition including a material changing an expression or an activity of FOG2 or USH protein, as an active ingredient.

In accordance with another still yet further object of the present invention, there is provided a use of a material changing an expression or an activity of FOG2 or USH protein, in preparation of an insulin signaling regulating composition.

### [Advantageous Effects]

The inventors found using Drosophila as a model system that conserved miRNA, miR-8, regulates body size by targeting u-shaped (ush) in fat body cells. Further, they confirmed that a human homolog (miR-200) of drosophila miR-8 (miRNA) targets a gene of FOG2. Also, they discovered that miR-8 and USH of drosophila are conserved also in mammals, and a human homolog of USH, that is, FOG2, acts by directly binding to the regulatory subunit of PI3K. When in human cancer cell lines, expression of miR-200 was inhibited, or FOG2 was expressed, it was found that PI3K activity facilitating cell growth was reduced. Thus, miR-200 and FOG2 may be usefully used in screening of an insulin signaling regulator such as an anti-cancer agent.

### [Description of Drawings]

FiGs. 1 to 4 show the comparison between miR-8 null flies (mir-8□2) and wild-type flies in phenotypes of female and male adults (see FIG. 1), body weight (see FIG. 2), pupariation and emergence (see FIG. 3), and wing cell size (see FIG. 4).
FiGs. 5 and 6 were obtained as follows: in miR-8 null flies, the activities and gene expression of proteins related to insulin signaling were determined by western blotting (see FIG. 5); and an expression level of 4EBP was determined through Q-PCR in miR-8 null larvae (see FIG. 6).
FIGs. 7 and 8 were obtained as follows: in the fat body, cuticle and brain tissues of the third-stage larvae (at 96 hr after egg-laying) of mir-8 enhancer trap GAL4 line drosophila (mir-8 gal4), the spatial expression pattern of miR-8 through immuno-staining was determined (see FIG. 7); and in the total larva, fat body, gut, brain and cuticle, the miR-8 expression level was determined through northern blotting (see FIG. 8).
FIGs. 9 and 10 show the comparison of body weight among mir8 CgG4>mir8, mir8 pplG4 and wild-type flies, and the comparison of body weight among mir8 elavG4, and mir8 elavG4>mir8.
FIG. 11 shows the homology between human miR-200 family miRNAs and drosophila miR-8.
FIGs. 12 and 13 show the confirmation that in human cell lines, MCF7, miR-200 family promotes cell proliferation (see FIG. 12); and the schematic view of an algorithm applied to selection of a target gene and ortholog through a target prediction program (see FIG. 13).
FIGs. 13 and 14 were obtained as follows: target gene candidates of drosophila miR-8 and human miR-200 miRNAs were selected using miRanda, miTarget, microT, PicTar and Target Scan (see FIG. 18); and 7 gene pairs that respond both to miR-8 and to mir-200 family miRNAs were identified by applying 3'UTR regions of the gene candidates to a luciferase expression system (see FIG. 14).
FIGs. 16 to 18 were obtained as follows: body weights of transgenic flies of mir8 Cg>ush i, mir8 Cg>Lap1 i, mir8 Cg>Ced-12 i, mir8 Cg>CG8445 i, mir8 Gg>CG12333 i and mir8 Cg>atro i were measured (see FIG. 16); whether each gene in the flies was knocked down was determined by quantitative RT-PCR (see FIG. 17); and body sizes of flies of w1118, w1118ush1518, mir-8□2 and mir-8□2ush1518 were measured (see FiG. 18).
FiGs. 19 to 21 were obtained as follows: whether the mRNA expression level of USH (selected as a target gene of mi-8) in miR-8 null flies was in actuality increased was determined by quantitative RT-PCR (see FIG. 19); whether the expression of USH protein was in actuality increased was determined by western blotting (see FIG. 20); and the regulation of miR-8 on ush 3'UTR's miR target site was determined by measuring luciferase activity through introduction of a mutation into the miR-8 target site (see FIG. 21).
FIG. 22 shows the measurement results of body size and wing cell number of drosophila when in drosophila fat body, insulin signaling was suppressed.
FIGs. 23 and 24 were obtained as follows: whether insulin signaling is defective in the fat body of miR-8 null flies was determined by observing PI3K activity and nuclear localization of FOXO in the fat body of miR-8 null flies (see FIG. 23); and in fat body tissues of wild-type larvae or miR-8 null larvae, JNK and Akt phosphorylation levels were determined by western blotting (see FIG. 24).
FIG. 25 shows the result when in the mosaic fat cells over-expressing miR-8, PI3K activity and cell size were observed through immunostaining.

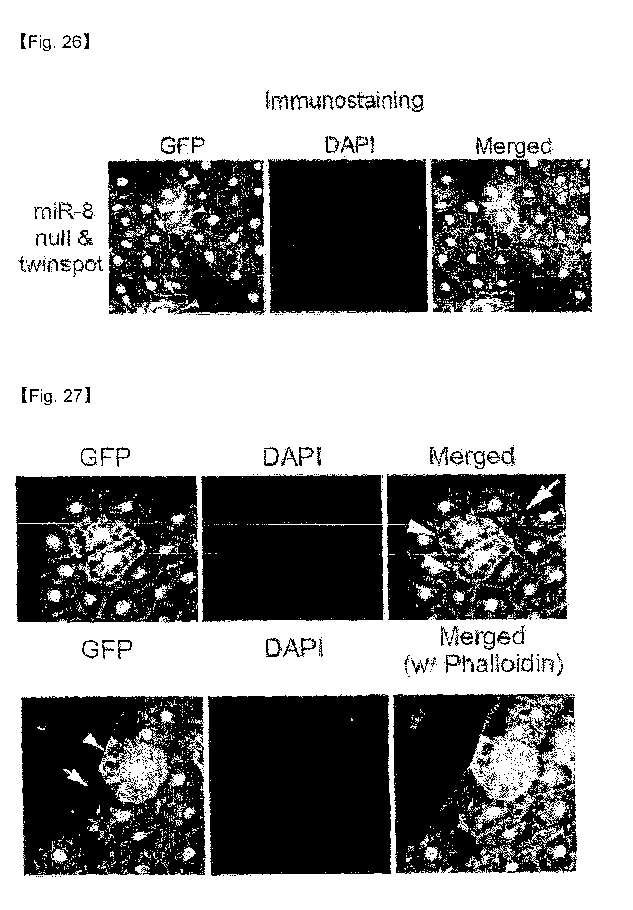

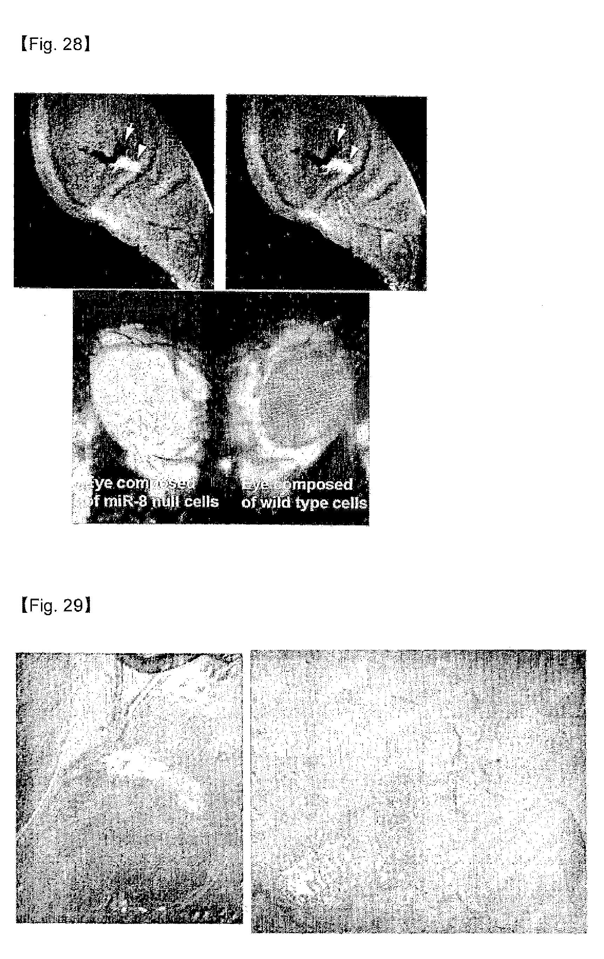

FIGs. 26 to 29 were obtained as follows: through twin-spot analysis (mitotic clone analysis), it was determined that a twin-spot of miR-8 null clones is more slowly generated than wild-type clones (arrow), and miR-8 null clones shows a lower DNA level than wild-type clones (see FIG. 26); in fat body cells over-expressing USH and wild-type cells, PI3K activity was determined (see FIG. 27); through twin-spot analysis in the wing or eye disc, it was determined whether the growth of the organs was inhibited when miR-8 null clones were generated (see FIG. 28); and in wing precursor cells or the eye disc of drosophila larvae, USH expression was determined (see FIG. 29).
FIGs. 30 to 34 were obtained as follows: in USH over-expressing cells, a cell size (see FIG. 30), a PI3K activity (see FIG. 31) and a nuclear FOXO signal (see FIG. 32) were determined through immune-staining; and in USH knockdown mosaic fat cells, a PI3K activity (see FIG. 33) and a nuclear FOXO signal (see FIG. 34) were determined.
FIG. 35 shows the result when it was determined whether insulin signaling in the fat body of miR-8 null larvae was suppressed by an increase in ush expression of fat body cells of CgG4, mir8 CgG4, mir8 CgG4>mir8 and mir8 Cg>ush i flies, through quantitative RT-PCR on FOXO target genes Inr and step mRNA.
FIGs. 36 and 37 show the detection result of a predicted binding site for miR-8 in ush mRNA (see FIG. 36), and the detection result of a predicted binding site for mi-200 family miRNAs in FOG2 (see FIG. 37).
FIG. 38 shows the result when the regulation of miR-200 miRNAs was determined through measurement of luciferase activity, in a case where one (FOG2 m1, FOG2 m2, FOG2 m3), two ((FOG2 m12, FOG2 m23) or three (FOG2 m123) mutants were introduced into three miR-200 target sites of FOG2 3'UTR.
FIG. 39 shows a correlation between the expression of FOG2 protein and miR-200c cluster miRNAs in human cell lines derived from different organs, and an expression of FOG2 protein and miR-200 family miRNAs in the cell lines, which were measured by western blotting and northern blotting, respectively.

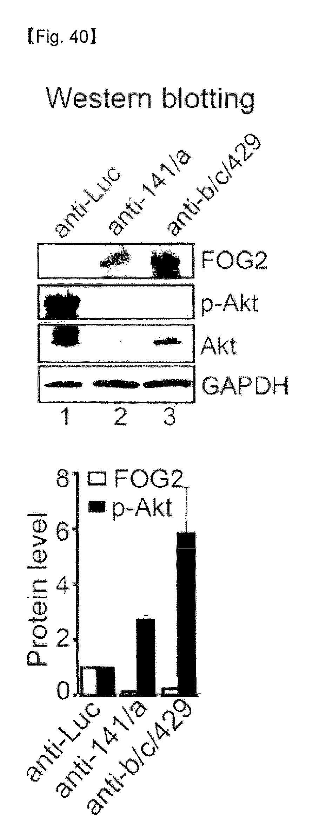

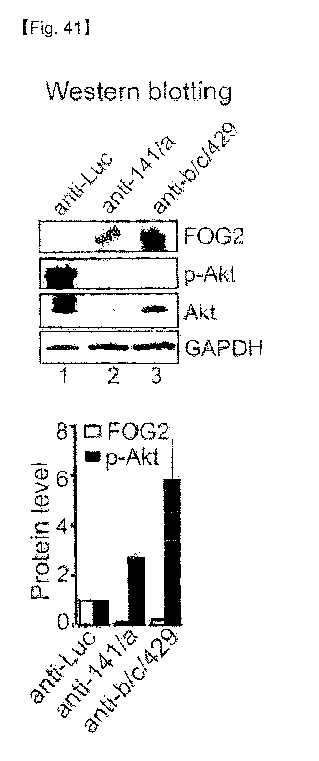

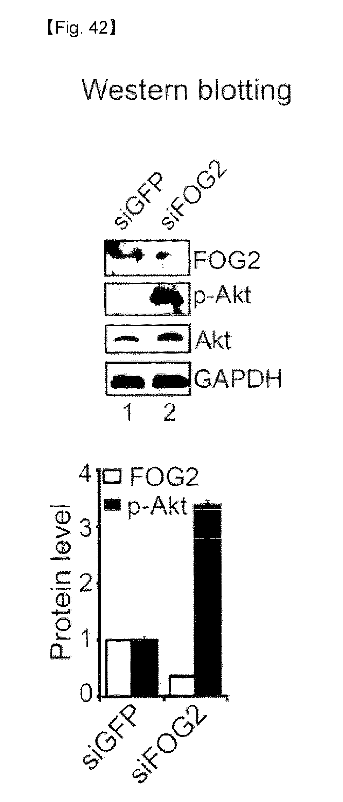

FIGs. 40 to 42 were obtained as follows: when miR-141/200a or miR-200b/c/429 was transfected into liver hepatocellular carcinoma Huh7 cells, an expression level of FOG and a phosphorylation level of Akt were determined through western blotting (see FIG. 40); when miR-141/200a or miR-200b/c/429 was treated with 2'-O-methyl oligonuclelotides antisense in pancreatic cancer cell lines, AsPC1 cells, an expression level of FOG and a phosphorylation level of Akt were determined through western blotting (see FIG. 41); when FAO cell lines were treated with siFOG2, an expression level of FOG and a phosphorylation level of Akt were determined through western blotting (see FIG. 42).
FIG. 43 shows the effect of FOG2 on PI3K activity, which was obtained through immune complex kinase assay after Hep3B cell lines were transfected with pCK-flag or pCK-FOG2.

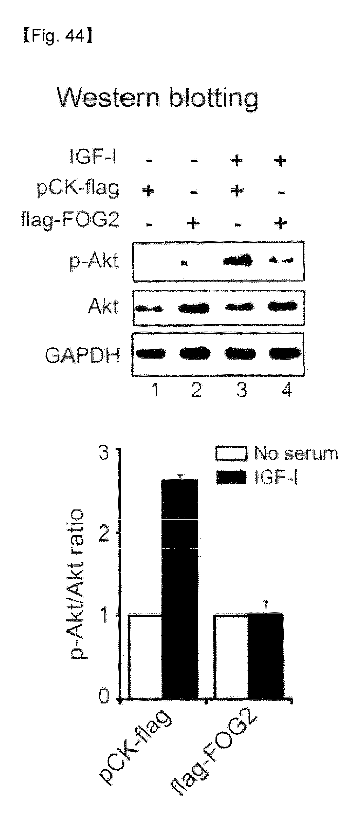

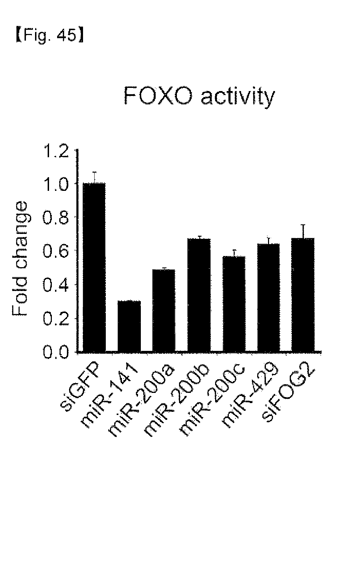

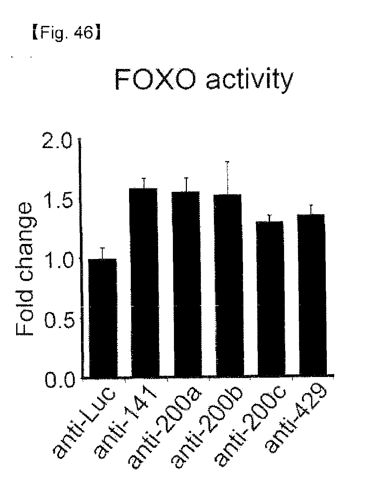

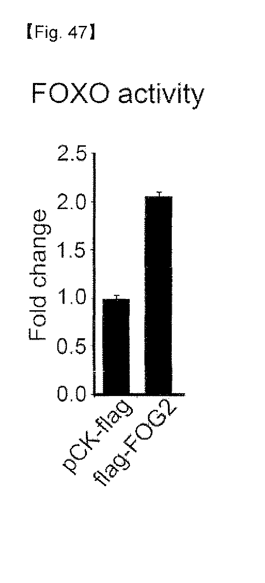

FIGS. 44 to 47 were obtained as follows: after Hep3B cell lines were transfected with pCK-flag or pCK-FOG2, a phosphorylation level of Akt, according to treatment with IGF-1 or FOG2, was determined through western blotting (see FIG. 44); in cells transfected with a luciferase reporter plasmid (pFK1tk-luc) containing eight FOXO binding sites, a change in the activity of luciferase according to transduction of miR-200 miRNAs was measured (see FIG. 45); according to transduction of complementary oligonucleotide, a change in the activity of luciferase was measured (see FIG. 46); and when FOG2 was over-expressed in Hep3B cell lines, a change in the activity of luciferase was measured (see FIG. 47).
FIGs. 48 and 49 show the measurement results of cell viability according to introduction of miR-200 family miRNAs (see FIG. 48) and introduction of oligonucleotide complementary to miR-200 family miRNAs (see FIG. 49) in Hep3B cells, which were obtained through MTT (3-(4,5-dimethylthiazol-2-yl)-2,5-diphenyltetrazolium bromide) assay.
FIG. 50 shows the result when it was determined whether IRS-1/p85α pa110 complex was formed according to expression or non-expression of FOG2 in Hep3B cell lines, through immunoprecipitation assay.
FIGs. 51 and 52 were obtained as follows: in the nucleus and cytoplasm fraction in Hela or PANC1 cells, expression of FOG2 was determined through western blotting (see FIG. 51); and in HepG2 cells, localization of FOG2 was determined through immunostaining (see FIG. 52).
FIGs. 53 and 54 show the result when it was determined whether FOG2 colocalizes together with p85α in PANC1 cells, through immunoprecipitation assay.
FiG. 55 shows FOG2 mutant bound to p85α, which was determined through immunoprecipitation assay when mutants of FOG2 and p85α were co-expressed in human cell lines derived from different tissues.
FIGs 56 to 58 were obtained as follows: when PANC1 cells were treated with mutant FOG2_1-412, mutant FOG2_413-789 or mutant FOG2_802-1151, PI3K activity according to an increase of an FOG2 expression level was measured (see FIG. 56); it was determined whether GST-fused recombinant p85α protein is directly bound to mutant FOG2_413-789 or mutant FOG2_802-1151 through in vitro binding assay (see FIG. 57); and when FOG2_413-789 or mutant FOG2_802-1151 was added to the immunoprecipitated PI3K complex, PI3K activity was determined (see FIG. 58).
FIGs. 59 and 60 show the result of immunoprecipitation assay, in which drosophila USH physically interacts with drosophila p60 (dp60, drosophila ortholog of p85α) when dp60 and USH were co-expressed in human HEK293T cells (in FIG. 59, IP for USH, and in FIG. 60, IP for dp60).
FIG. 61 is a schematic view showing an intracellular signaling process of USH/FOG2.

### [Best Mode]

Hereinafter, the present invention will be described in detail.

In order to achieve the above objects, the present invention provides a method of screening an insulin signaling regulator, the method including the steps of:
1) treating cell lines expressing miR-200 family miRNAs or miR-8 miRNA with a testing compound;
2) measuring an expression level or an activity of FOG2 or USH protein in cells treated with the testing compound in the step 1); and
3) identifying the testing compound by which the expression or the activity of the FOG2 or USH protein in the cells in the step 1) has been changed as compared to a control.

Also, the present invention provides a method of screening an insulin signaling regulator, the method including the steps of:
1) treating cell lines expressing FOG2 or USH with a testing compound;
2) measuring an expression level or an activity of FOG2 or USH protein in cells in the step 1); and
3) identifying the testing compound by which the expression or the activity of the FOG2 or USH protein in the cells in the step 1) has been changed as compared to a control.

Also, the present invention provides a method of screening an insulin signaling regulator, the method including the steps of:
1) bringing FOG2 protein into contact with p85α protein in the presence of a testing compound;
2) measuring a degree of binding of the FOG2 protein to the p85α protein; and
3) identifying the testing compound by which the degree of binding of the FOG2 protein to the p85α protein in vitro in the step 2) has been changed as compared to a control not treated with the testing compound.

Also, the present invention provides a method of screening an insulin signaling regulator, the method including the steps of:
1) bringing USH protein into contact with dp60 protein in the presence of a testing compound;
2) measuring a degree of binding of the USH protein to the dp60 protein; and
3) identifying the testing compound by which the degree of binding of the USH protein to the dp60 (Drosophila p60) protein in vitro in the step 2) has been changed as compared to a control not treated with the testing compound.

Also, the present invention provides a method of screening an insulin signaling regulator, the method including the steps of:
1) treating cell lines expressing FOG2 protein with a testing compound;
2) measuring a degree of binding of FOG2 protein to p85α protein in cells treated with the testing compound in the step 1); and
3) identifying the testing compound by which the degree of binding of the FOG2 protein to the p85α protein in the cells in the step 1) has been changed as compared to a control not treated with the testing compound.

Also, the present invention provides a method of screening an insulin signaling regulator, the method including the steps of:
1) treating cell lines expressing USH protein with a testing compound;
2) measuring a degree of binding of USH protein to dp60 protein in cells treated with the testing compound in the step 1); and
3) identifying the testing compound by which the degree of binding of the USH protein to the dp60 protein in the cells in the step 1) has been changed as compared to a control not treated with the testing compound.

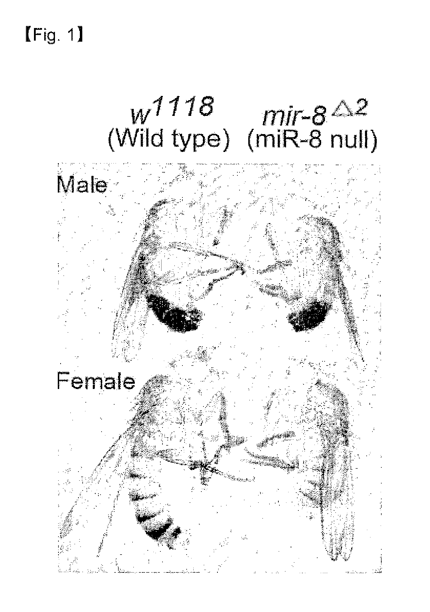

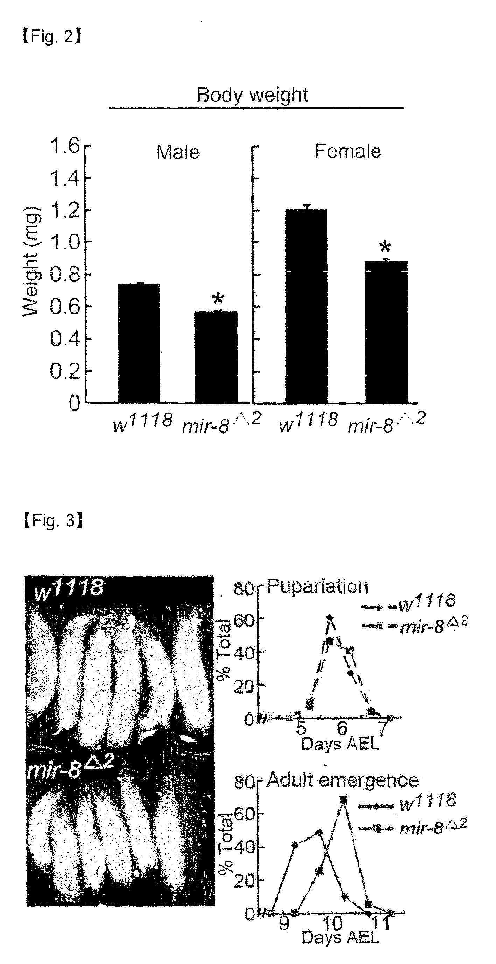

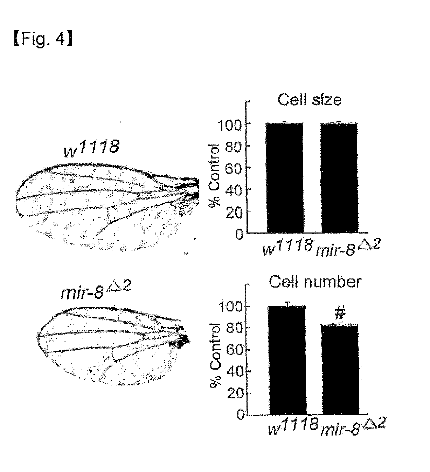

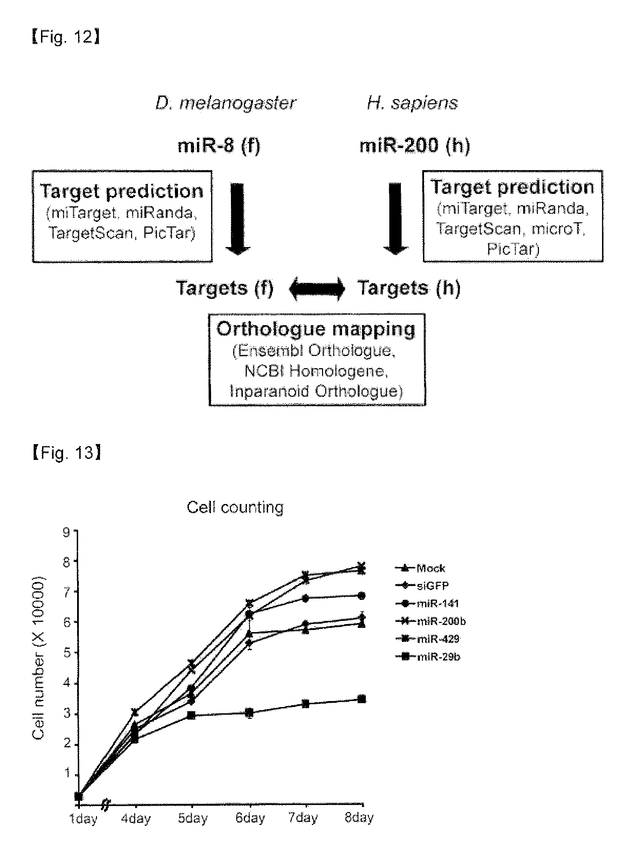

As a sole homolog of miR-200 family promoting proliferation of human cancer cells (see FIG. 13), miR-8 is known (Ibanez-Ventoso et al., 2008). Thus, the inventors have tried to discover the biological function of miR-200 by using drosophila. It is generally known that reduced body size in insects is caused by either slow larval growth, precocious early pupariation (pupal formation) that shortens the larval growth period, or both (Colombaniet al., 2003; Edgar, 2006; Mirth and Riddiford, 2007). The inventors found that miR-8 null flies exhibit a significantly small body size and a significantly small body weight, and miR-8 null larvae exhibit a significantly small body volume (see FIGS. 1, 2 and 3). Also, it was found that the small body size of miR-8 null flies is likely to be caused by slower growth during the larval period rather than by precocious pupariation, and also caused by decreased cell number rather than reduced cell size (see FIGs. 3 and 4).

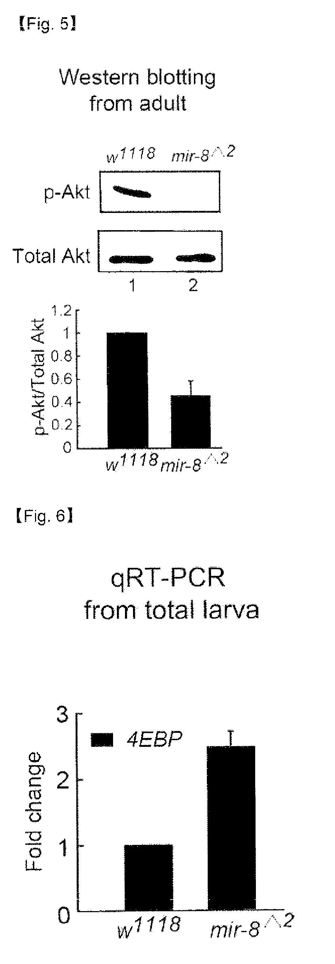

Also, in order to understand why miR-8 null flies grow slowly, the inventors examined the activities of the proteins involved in insulin signaling in the miR-8 null flies. As a result, it was found that that insulin signaling was significantly reduced in the miR-8 null flies (see FIGs. 5 and 6).

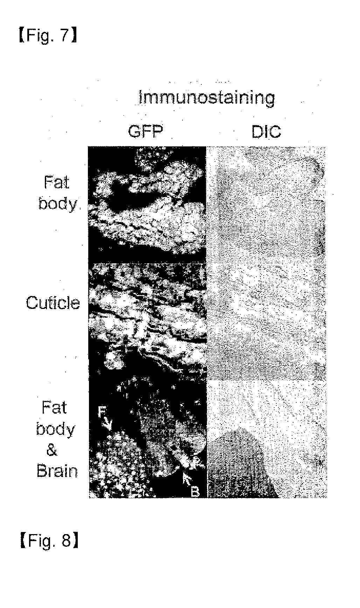

The inventors examined the spatial expression pattern of miR-8 in larvae. As a result, they found a high miR-8 expression level in the fat body of larvae (see FIGs. 7 and 8).

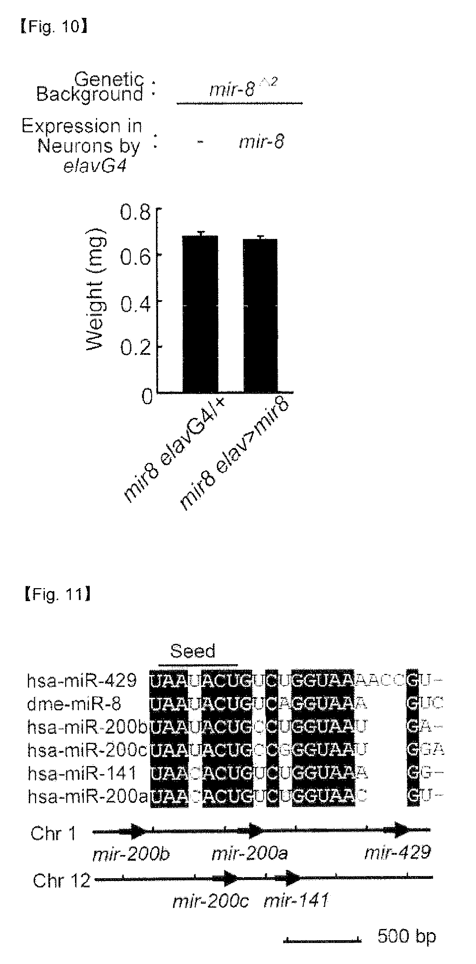

Recent studies suggested that Drosophila fat body may be an important organ in the control of energy metabolism and growth (Colombani et al., 2005; Colombani et al., 2003; Edgar, 2006; Leopold, P, and Perrimon, N 2007, Nature 450, 186-188). The inventors confirmed that miR-8 expression in the larval fat body is critical for body size control of drosophila, and the function of miR-8 in the fat body is spatio-temporally specifically distinct from neuronal cells (see FIGs. 10 and 11).

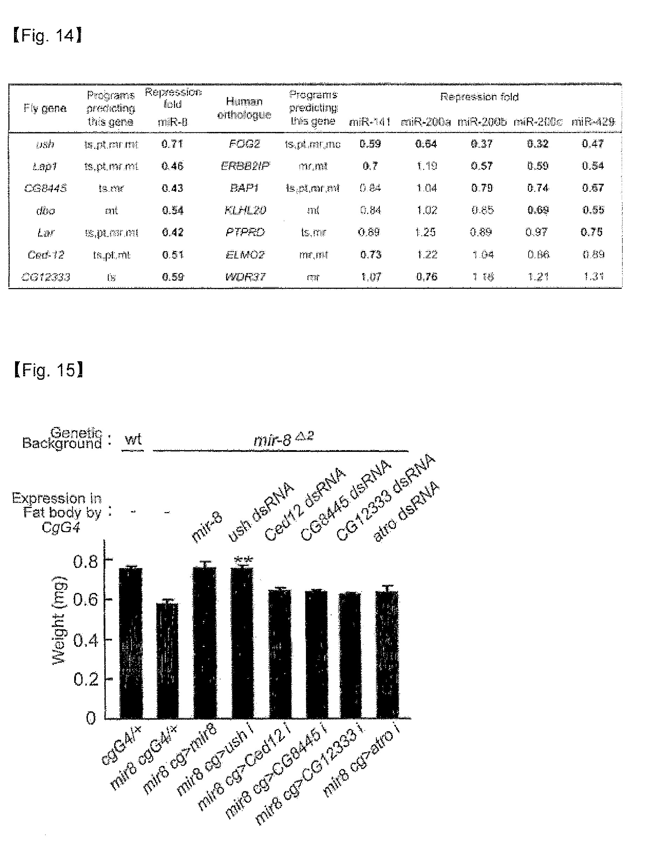

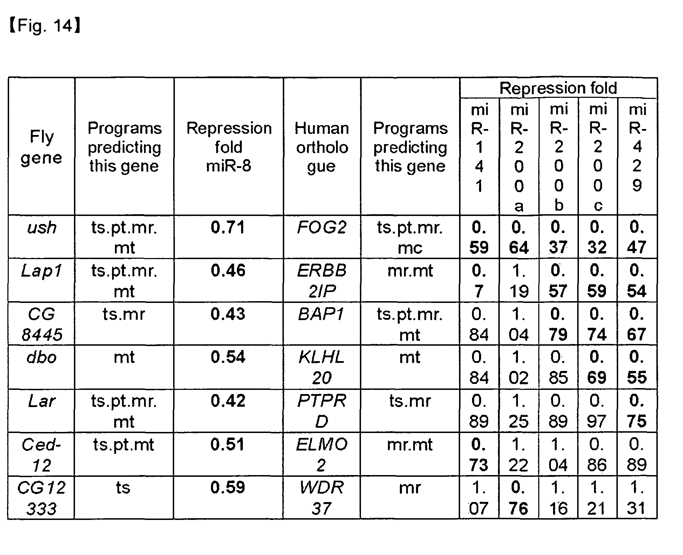

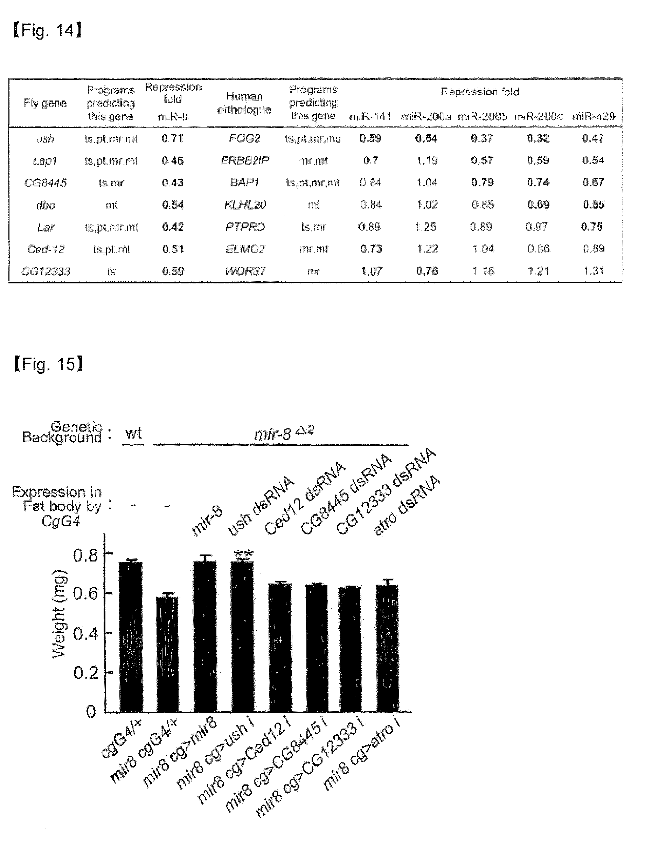

To further understand the molecular function of miR-8, the inventors set out to identify miR-8 target genes responsible for body size control. Because miR-8 is a highly conserved miRNA and because the human homologs of miR-8 promote cell proliferation in human cells (see FIG. 6), the inventors assumed that the target genes of miR-8 are related to cell proliferation phenotype. The inventors listed target gene candidates of both fly miR-8 and human miR-200 miRNAs by using a target prediction program (see FIG. 14). The listed candidates were divided into two groups of fly genes and human genes. From the target candidate list, the inventors selected, as target genes, drosophila genes, ush, Lap1, CG8445, dbo, Lar, Ced-12, CG12333, cbt, Scr, pan, pnut, Spri, Shc, Rassf and CG5608, that are known as tumor suppressors or negative regulators of cell proliferation in at least one species, and the respective human orthologs of the drosophila genes, FOG2, SLEPT7, RIN2, SHC1, HOXB5, KLF11, TCF7L1, ERBB2IP, ELMO2, BAP1, W DR37, KLHL20, PTPRD, RASSF2 and VAC14. The Drosophila gene u-shaped (ush) and its human homolog Friend of GATA 2 (FOG2, also known as ZFPM2) were the most frequency predicted gene pair conserved in both species.

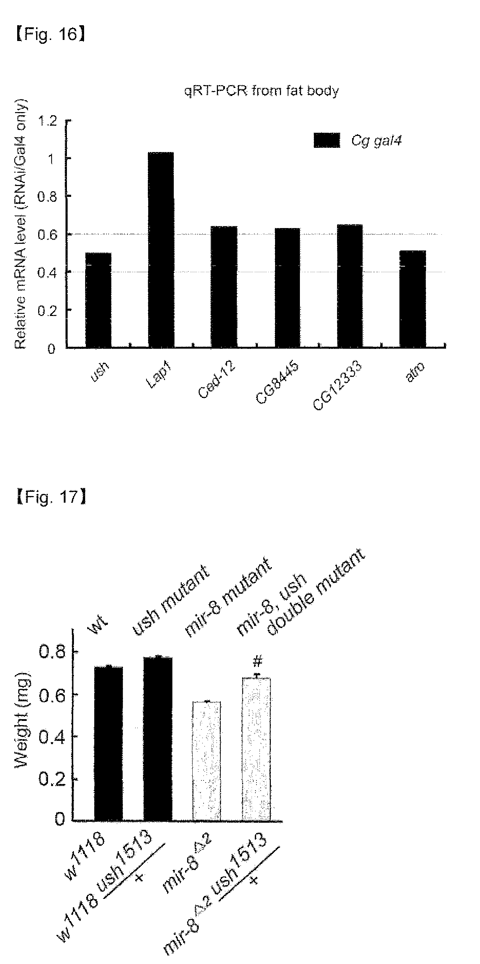

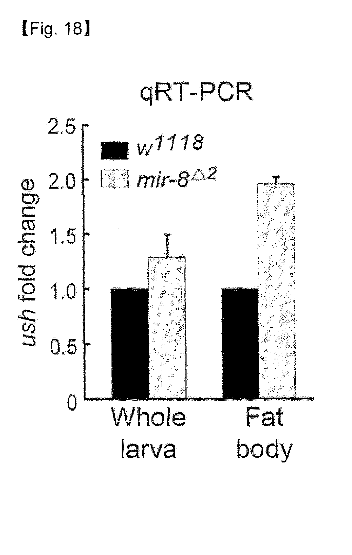

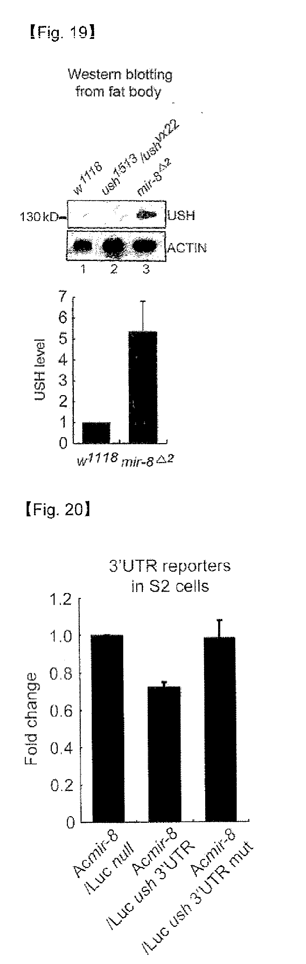

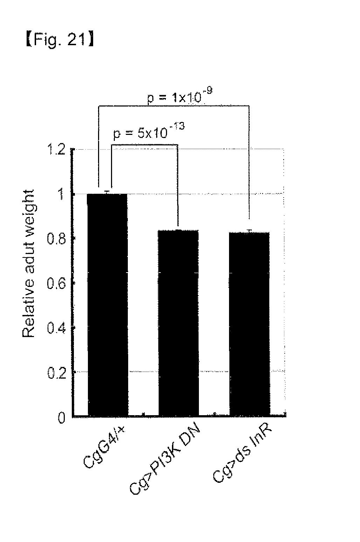

From among the listed target genes, the inventors identified, as targets of miR-8 and miR-200 family miRNAs, 7 gene pairs that respond both to miR-8 and to miR-200 family miRNAs in fly and human cells, respectively (see FIG. 15). Then, they confirmed that a target gene of miR-8 miRNA is ush, the function of miR-8 in the prevention of neurodegeneration (Karres et al., 2007) is separate from its function in body growth, not only spatially but also at the molecular level (see FIG. 16), and USH inhibits the body growth (see FIG. 18). Thus, it was found that miR-8 inhibits ush in the fat body (see FIG. 19). Thus, it was found that ush is more strongly suppressed in the fat body than in other body parts. Notably, USH protein levels are more dramatically reduced than the mRNA levels (see FIG. 20), indicating that miR-8 represses USH expression by both mRNA destabilization and translational inhibition (Karres et al., 2007). Also, the inventors confirmed that suppression of USH expression is mediated through direct binding of miR-8 to the predicted target site of USH (see FIG. 21). Further, putative target sites for miR-8 were found in all Drosophilid species examined, including distant species such as D. virilis and D. grimshawi. Thus, they found that ush is an authentic target of miR-8.

Several reports have suggested that insulin signaling in the larval fat body controls organismal growth in a non-cell-autonomous manner (Britton, JS et al., 2002, Dev Cell 2, 239-249; Colombani et al., 2005).

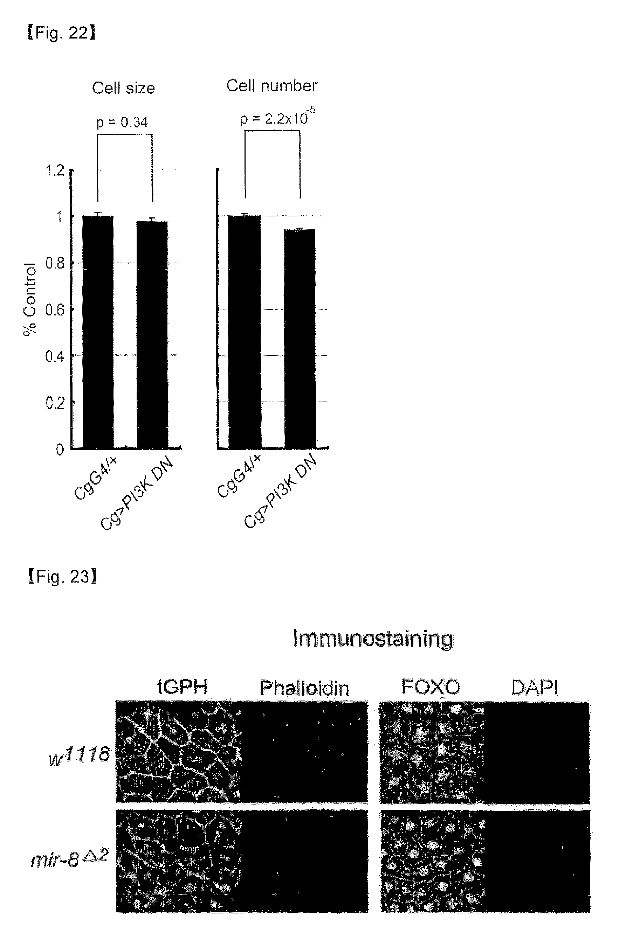

The inventors observed that suppression of insulin signaling in the fat body of flies yielded smaller flies and fewer cells in the wing (see FIG. 22) in the same manner as those in miR-8 null flies (see FIG. 4). From this result, they assumed that USH suppresses body growth by inhibiting insulin signaling. Then, they investigated whether inhibition of insulin signaling in the larval fat body suppresses the growth of flies. As a result, it was found that insulin signaling was specifically disrupted in the fat bodies of miR-8 null larvae (see FiGs, 18, 23 and 24).

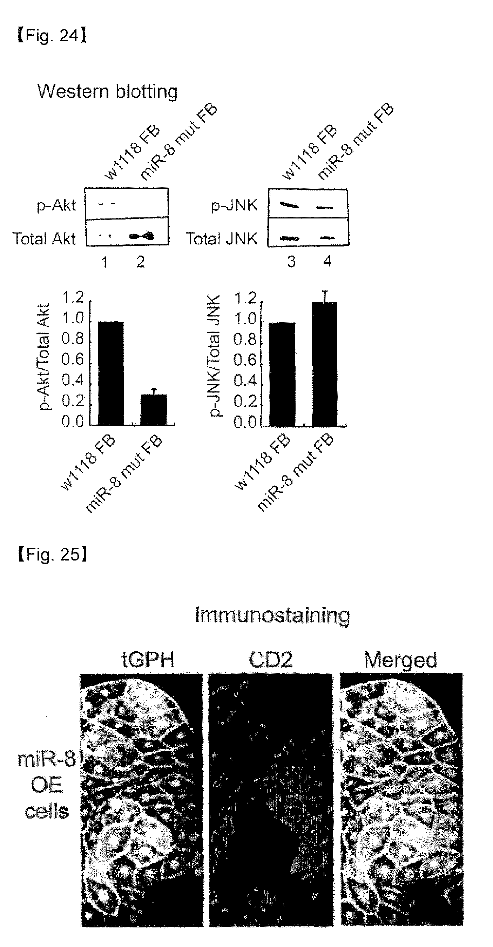

Also, the inventors more precisely analyzed miR-8's function in fat cells. As a result, they confirmed that miR-8 activates PI3K in a cell-autonomous manner, thereby promoting cell growth (see FIG. 25), and also the effect of miR-8 on the cell growth depends on the type of expressed tissues (see FIGs. 20, 28, and 29).

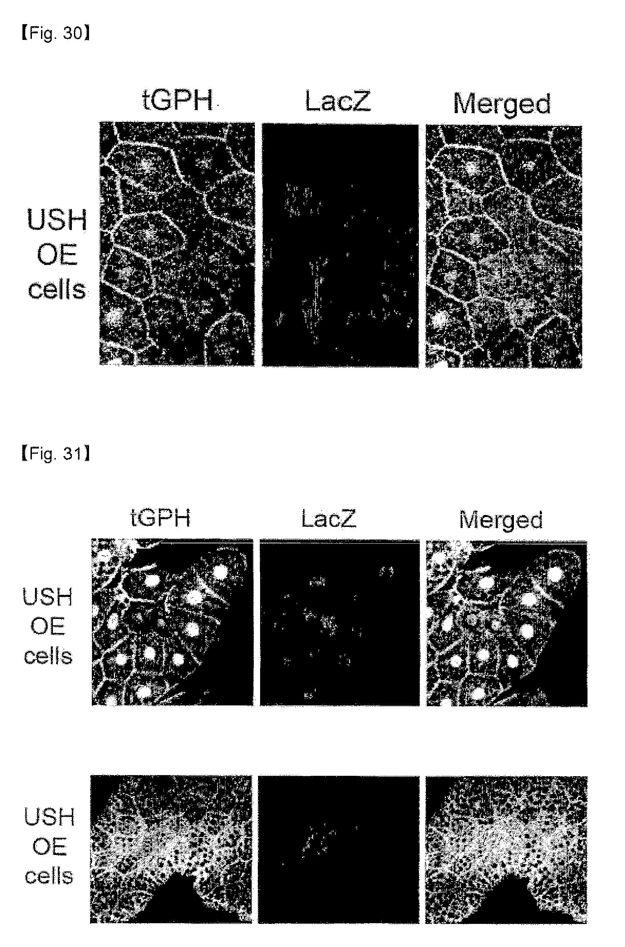

Also, the inventors investigated whether USH negatively regulates insulin signaling. As a result, they found that USH inhibits insulin signaling upstream of or in parallel with PI3K in a cell-autonomous manner (see FIGs. 30, 31, 32, 33 and 34).

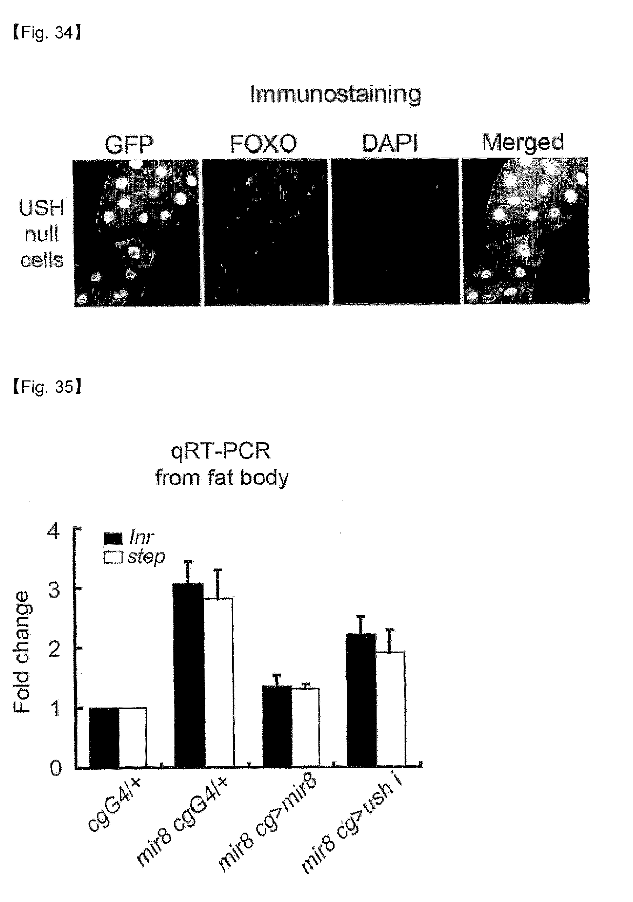

Excessive insulin signaling is known to reduce the levels of insulin receptor (Inr) and cytohesin Steppke (step) through negative feedback by FOXO (Fuss, B et al.. 2006, Nature 444, 945-948; Puig, O, and Tjian, R 2005, Genes Dev 19, 2435-2446). The inventors examined whether reduced insulin signaling caused by the absence of miR-8 could be rescued by knockdown of USH. As a result, it was found that the defect of insulin signaling in the fat body of miR-8 null larvae is at least partially attributable to elevated ush levels (see FIG. 35).

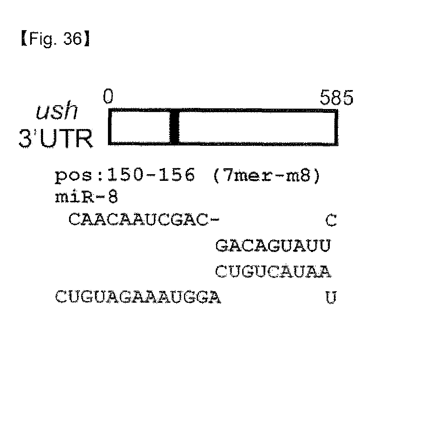

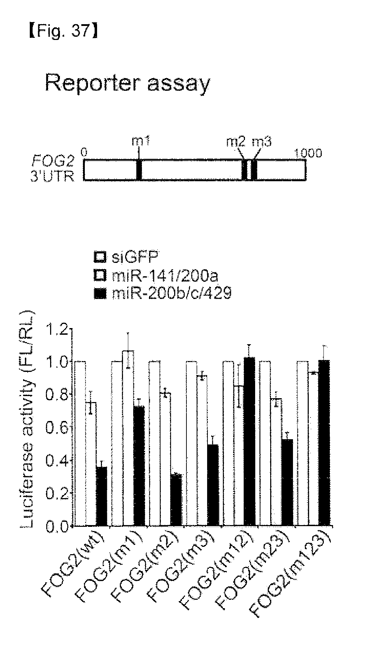

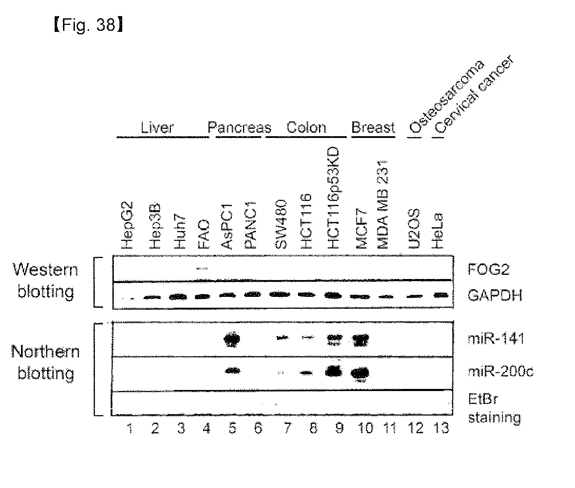

According to the result of detection of miRNA target genes, the ush mRNA has one predicted binding site for miR-8, whereas the mammalian ortholog of ush, FOG2, has at least three predicted sites for miR-200 family miRNAs (see FIGS. 36 and 37). The inventors confirmed that human miR-200 family miRNAs regulate the expression by directly binding to 3'UTR of fog2 mRNA in the same manner as drosophila miR-8 miRNA regulates the expression by directly binding to 3'UTR of ush mRNA (see FIG. 38).

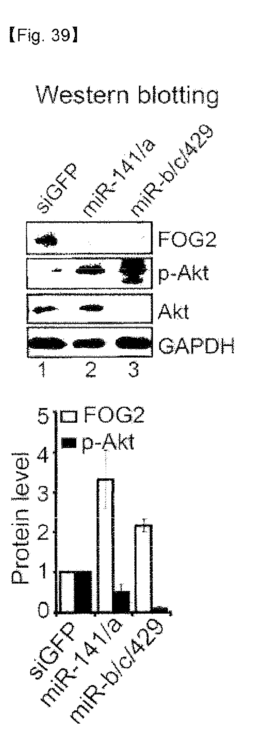

FOG2 is expressed in heart, brain, testicle, liver, lung and skeletal muscle (Holmes, M et al., 1999, J Biol Chem 274, 23491-23498; Lu, JR et al., 1999,/Mol Cell Biol 19, 4495-4502; Svensson, EC et al., 1999, Proc Natl Acad Sci U S A 96, 956-961; Tevosian, SG et al., 1999, Proc Natl Acad Sci U S A 96, 950-955). Despite its relatively broad expression in adult tissues, little is known about the function of FOG2 beyond its role in embryonic heart development (Fossett, N, and Schulz, RA 2001, Trends Cardiovasc Med 11, 185-190). Meanwhile, the miR-200 miRNAs have also been reported to be expressed in various adult organs, including pituitary gland, thyroid, pancreatic islet, testes, prostate, ovary, breast, and liver (Landgraf, P et al., 2007, Cell 129, 1401-1414). The inventors looked for a correlation between the expression of FOG2 protein and miR-200c cluster miRNAs in human cell lines derived from different organs. As a result, there is generally a negative correlation between miR-200c cluster members and FOG2 in expression level (see FIG. 39).

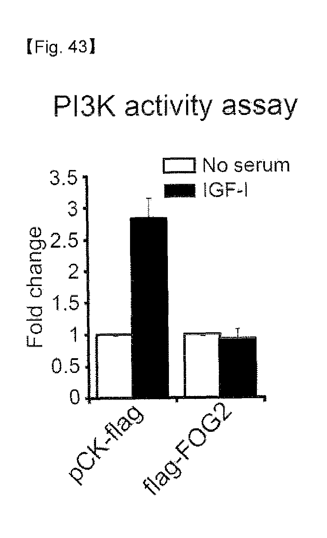

Also, the inventors confirmed that FOG2 is in actuality reduced by miR-200 miRNAs (see FIGs. 40, 41 and 39), and that human miR-200 miRNAs have a conserved role in the modulation of insulin signaling, as in the case of fly miR-8 miRNA (see FIGs. 40, 41 and 42). Also, it was confirmed that FOG2 suppresses PI3K (see FIG. 43), and phosphorylation of Akt is reduced by FOG2 (see FIG. 44). Akt represses FOXO activity. When the effect of miR-200 miRNAs on the downstream transducers of Akt was analyzed, it was found that FOXO activity is reduced by miR-200 miRNAs, and FOXO activity is increased by FOG2 (see FIG. 45, 46 and 47).

The inventors investigated whether miR-200 specifically affects the insulin signaling pathway. As a result, it was found that miR-200 specifically modulates PI3K-Akt-FOXO signaling (see FIGs. 44 and 45).

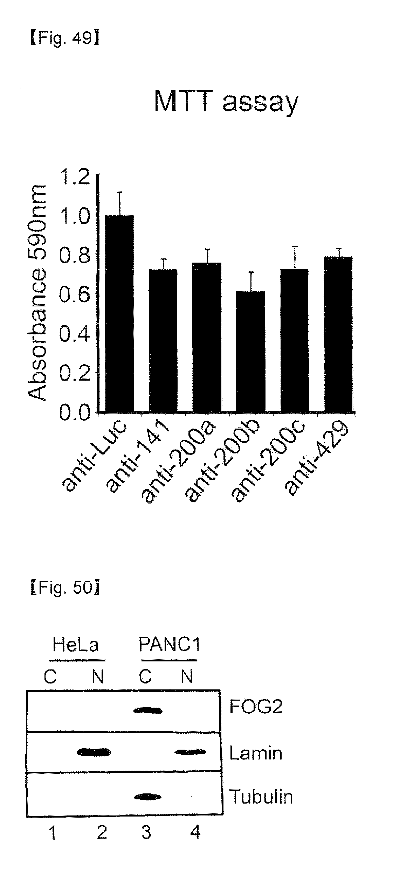

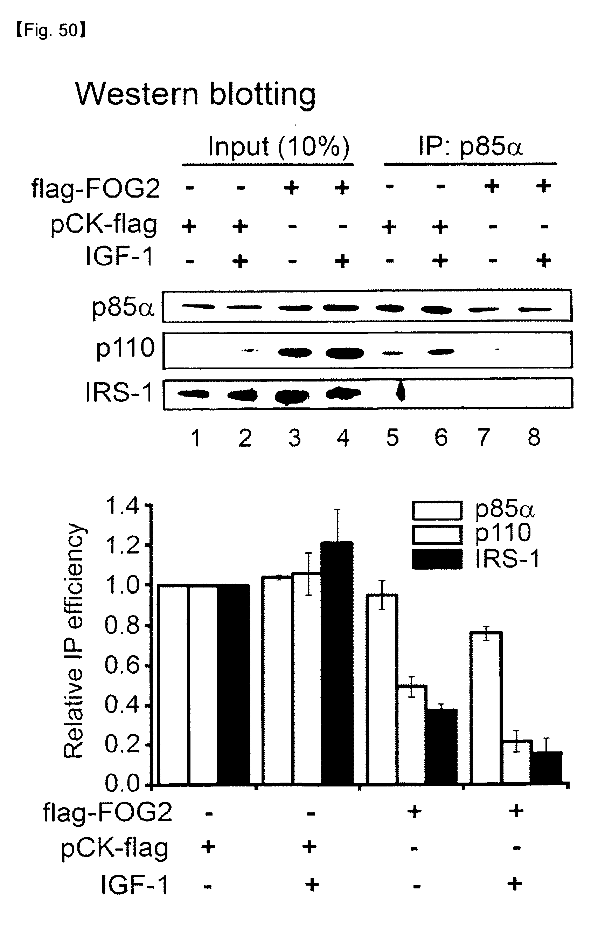

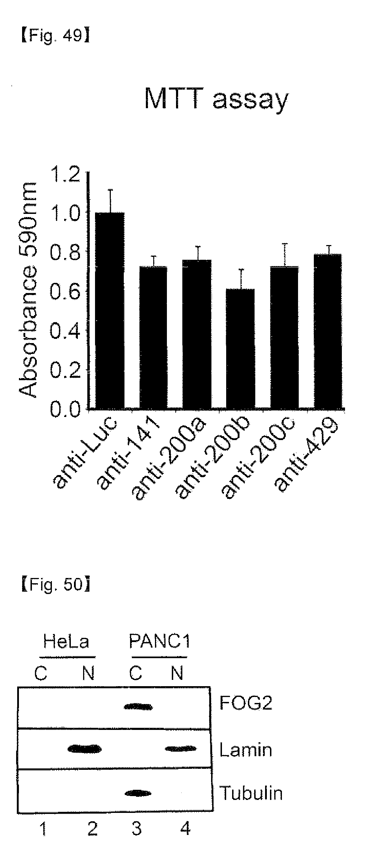

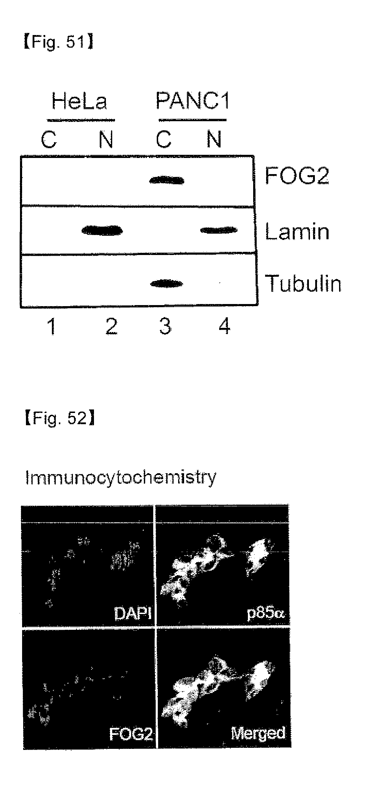

Simulation of PI3K and AKT is known to facilitate cell proliferation, and antagonize apoptosis (Pollak, 2008). Thus, consistently, the introduction of miR-200 miRNAs increased cell viability (see FIG. 50), whereas the introduction of miRNA inhibitors produced the opposite effect (see FIG. 50). Also, through investigation of the action mechanism of FOG2, it was found that FOG2 acts as a negative regulator of PI3K by interfering with the formation of an IRS-1/p85α/p110 complex (see FIGs. 51 and 52).

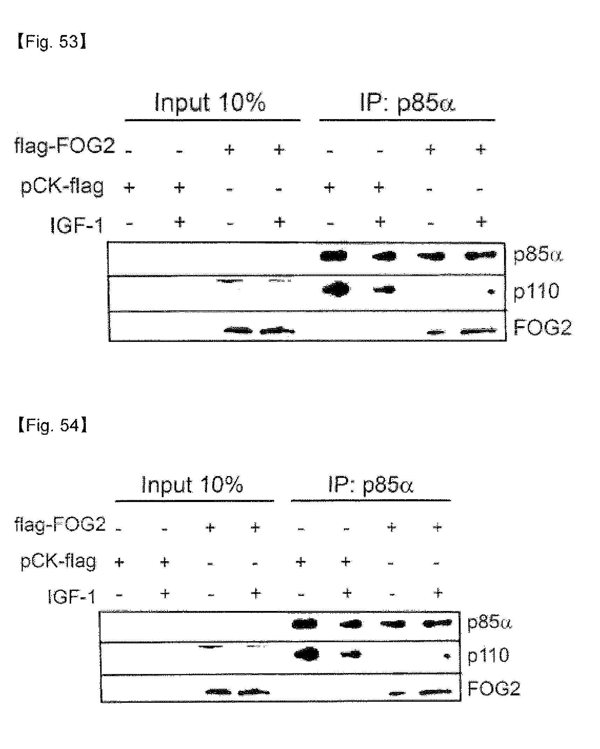

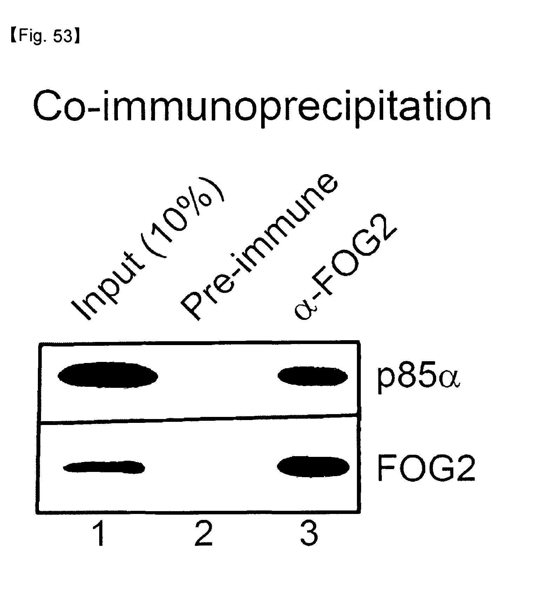

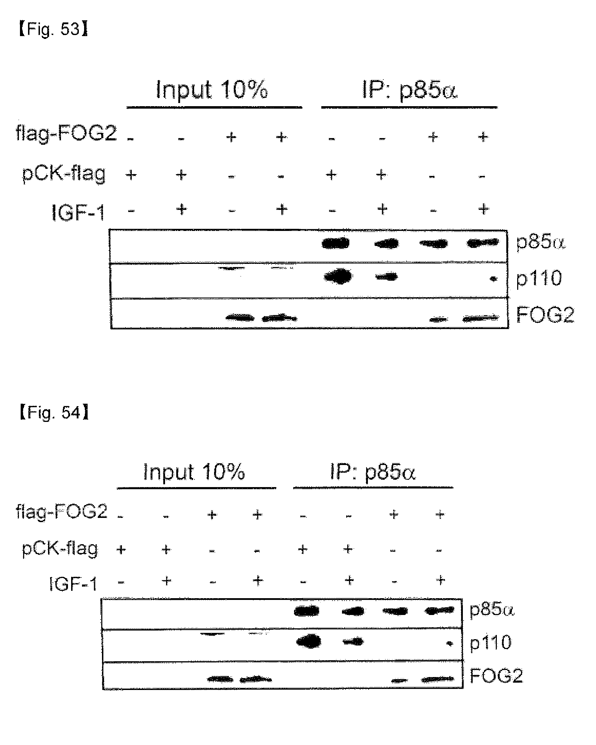

Although FOG2 is thought to be a nuclear transcriptional co-regulator, several studies have reported that FOG2 also localizes to the cytoplasm (Bielinska, M et al., 2005, Endocrinology 146, 397M 3984; Clugston, RD et al., 2008, Am J Physiol Lung Cell Mol Physiol294, L665-675). This indicates that FOG2 has a function within the cytoplasm (see FIGs. 53 and 54).

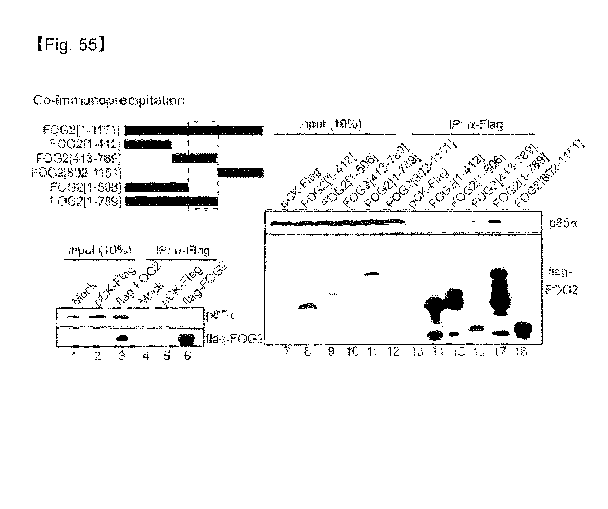

It was tested whether FOG2 interacts with PI3K. As a result, it was determined that FOG2 directly binds to p85α, the regulatory subunit of PI3K (see FIGs. 55 to 60).

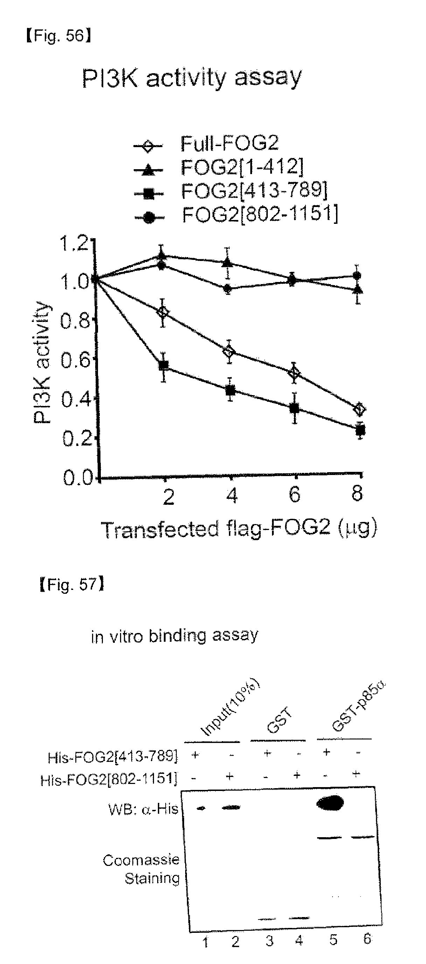

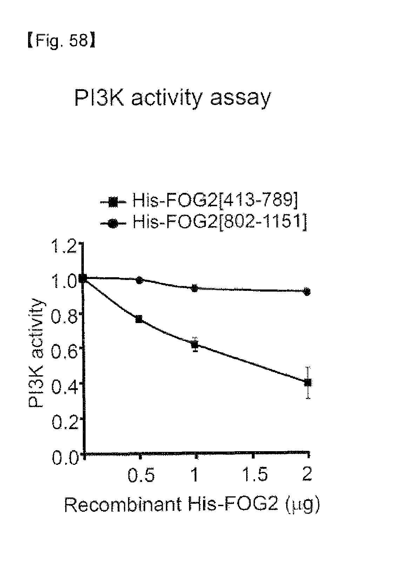

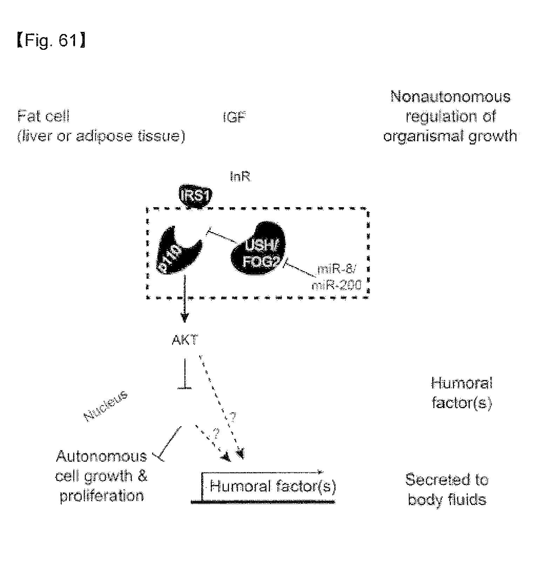

In order to determine FOG2's domain to be bound to p85α, the inventors generated several truncated mutants of FOG. As a result, it was confirmed that the middle region of FOG2 (507-789 aa) mediates the interaction with p85α (see FIG. 57), and has an important role of suppressing PI3K (see FIG. 58). This result suggests that direct binding of FOG2 to p83 leads to the inhibition of PI3K activity. Especially, the inventors found that drosophila USH physically interacted with drosophila p60 (dp60; the fly ortholog of p85α) when dp60 was co-expressed with USH in human HEK293T cells (see FIG. 61). From this result, it can be found that the action mechanism of USH/FOG2 may be conserved across the phyla (see FIG. 61).

It is preferable that the cell lines expressing miR-200 family miRNAs are human cell lines derived from the heart, brain, testicle, liver, lung and skeletal muscle tissues, and the cell lines expressing miR-8 miRNA are drosophila cell lines derived from fat body tissues, but the present invention is not limited thereto.

The measurement of the expression level of FOG2 or USH protein is preferably carried out by any one method selected from the group including western blotting, immunostaining, fluorescent staining, and reporter assay, and the measurement of bindings between FOG2 and p85α, and USH and dp60, and the measurement of the formation of a p85a/p110/IRS-1 complex are preferably carried out by co-immunoprecipitation assay, but the present invention is not limited thereto.

In the step 1), the testing compound is preferably any one selected from the group including
natural compounds, synthesized compounds, RNA, DNA, polypeptides, enzymes, proteins, ligands, antibodies, antigens, metabolites of bacteria or fungi, and bioactive molecules, whose anti-cancer agent enhancing effect is unknown to the person skilled in the art.

The activity of FOG2 or USH protein may be measured by any one method selected from the group including i ) a method of measuring activity of p85α/p110 complex; ii ) a method of measuring expression level of FOXO mRNA or protein; and iii ) a method of measuring cell growth and proliferation, and also, the activity of p85α/p110 complex (PI3K) may be measured by measuring kinase activity of PI3K or phosphorylation level of AKT protein, but the present invention is not limited thereto.

The binding between proteins may be measured by any one method selected from the group including SPR, protein chip, gel shift assay, immunoprecipitation assay, co-immunoassay, fluorescence immuno assay and radioimmuno assay, but the present invention is not limited thereto. Also, all methods for measuring binding of proteins, conventionally used in the art, may be used.

Also, the present invention provides an insulin signaling regulating composition including a material changing expression or activity of FOG2 or USH protein, as an active ingredient.

It is preferable that a material changing expression FOG2 or USH protein may be an expression inhibitor of FOG2 or USH protein, and the expression inhibitor of FOG2 or USH protein may be an antisense nucleotide or a small interfering RNA (siRNA) that complementarily binds to mRNA of fog2 or ush gene. Also, the material changing the activity of FOG2 or USH protein may be an activity inhibitor of FOG2 or USH protein, and the activity inhibitor of FOG2 or USH protein may be any one selected from the group including materials to be complementarily bound to FOG2 or USH protein such as cytotoxic compound, peptide, peptide mimetics and antibody, but the present invention it not limited thereto.

The siRNA includes a 15- to 30-mer sense sequence selected from base sequences of mRNA of gene (SEQ NO:42 or SEQ ID:15) coding FOG2 or USH, and an antisense sequence complementarily bound to the sense sequence. Herein, the sense sequence preferably includes 19 to 27 bases, and FOG2 protein expression inhibitor more preferably has a base sequence represented by SEQ ID:119, although not particularly limited thereto.

The antisense nucleotide is hybridized with a complimentary base sequence of DNA, immature-mRNA or mature mRNA as determined by Watson-Crick base pairing, and then, as a protein in DNA, interferes with the stream of genetic information. The antisense nucleotide has specificity for a target sequence. This characteristic allows it to be exceptionally multi-functional. The antisense nucleotide is a long chain of a monomer unit, and thus can be easily synthesized on a target RNA sequence. From many recent studies, it was proved that an antisense nucleotide is useful as a biochemical means for research of target protein (Rothenberg et al., J. Natl. Cancer inset., 81:1539-1544, 1999). Since the nucleotide synthesis field related to oligonucleotide chemical and enhanced cell adhesion, target binding affinity and nuclease resistance has been recently considerably advanced, an antisense nucleotide may be considered as a novel inhibitor.

The Peptide Mimetics are peptides or non-peptides that inhibit a binding domain of FOG2 or USH protein. They suppress activity of FOG2 or USH protein. Main residues of a non-hydrolyzed peptide analogue may be generated by using β-turn dipeptide core (Nagai et al. 1985,Tetrahedron Lett 26:647), keto-methylene pseudo peptides (Ewenson et al. 1986, J Med Chem 29:295; and Ewenson et al. 1985, in Peptides: Structure and Function (Proceedings of the 9th American Peptide Symposium) Pierce Chemical Co. Rockland, IL), azepine (Huffman et al. 1988, in Peptides: Chemistry and Biology, G.R. Marshall ed., ESCOM Publisher: Leiden, Netherlands), benzodiazepine (Freidinger et al. 1988, in Peptides; Chemistry and Biology, G.R. Marshall ed., ESCOM Publisher: Leiden, Netherlands), β-amine alcohol (Gordon et al. 1985, Biochem Biophys Res Commun 126:419) and substituted gamma lactam ring (Garveyet al., 1988 in Peptides: Chemistry and Biology, G.R. Marshell ed., ESCOM Publisher: Leiden, Netherlands).

The inventive composition includes the active ingredient in an amount of 0.0001 to 50 wt% with respect to the total weight of the composition.

The inventive composition may include, besides a material changing expression or activity of FOG2 or USH protein, at least one kind of active ingredient showing the same or similar function as that of the material.

The inventive composition, for administration, may be prepared by additionally including at least one kind of pharmaceutically acceptable carrier beside the above described active ingredient. As the pharmaceutically acceptable carrier, saline solution, sterilized water, Ringer's solution, buffered saline solution, dextrose solution, malto dextrine solution, glycerol, ethanol, liposome, and a mixture of one or more thereof may be used, and if necessary, other usual additives such as an anti-oxidant, buffer solution, bacteriostatic, etc. may be added. Also, a diluent, dispersion agent, surfactant, binder, and lubricant may be further added for formulation of injectable forms (such as aqueous solution, suspension, and emulsion), pills, capsules, granules or tablets. Also, the carrier may be used in combination with organ specific antibody or other ligands so that it can specifically act on an organ. Further, the composition may be formulated desirably according to each disease or component by using a proper method in the present field or a method disclosed in Remington's Pharmaceutical Science (updated version), Mack Publishing Company, Easton, Pa.

Nucleotide or nucleic acid, used in the present invention, may be prepared for administration of oral, topical, parenteral, intranasal, intravenous, intramuscular, subcutaneous, ocular, transdermal and routes. Preferably, nucleic acid or vector is used in an injectable form. Accordingly, especially for direct injection into a treatment area, it may be used in combination with any pharmaceutically acceptable vehicle for an injectable composition. The inventive composition may include a freeze-dried composition which can have an injectable solution composition according to addition of isotonic sterilized solution, dried sterilized water or proper saline solution. The direct injection of nucleic acid into tumor of a patient is advantageous in that therapeutic efficiency can be focused on infected tissues. The dosage of nucleic acid to be used may be adjusted according to various parameters, especially gene, vector, administration method in use, problematic disease or alternatively required treatment period. Also, the range of the dosage may vary according to body weight, age, sex, health condition, diet, administration time, administration method, excretion rate, and severity of disorder of a patient. The daily dosage ranges about from 0.01 to 12.5 mg/kg, and preferably from 1.25 to 2.5 mg/kg. Also, it is preferable to administer the composition once to several times a day.

The inventive insulin signaling regulating composition may be usefully used in molecular biological research of insulin signaling pathway, and prevention or treatment of diseases that can be caused by abnormal action of insulin signaling pathway, e.g., cancer, diabetes mellitus and obesity.

Further, the present invention provides a use of a material changing expression or activity of FOG2 or USH protein in the preparation of an insulin signaling regulating composition.

Preferably, the material changing expression of FOG2 or USH protein may be an expression inhibitor of FOG2 or USH protein, and the expression inhibitor of FOG2 or USH protein may be an antisense nucleotide or a small interfering RNA (siRNA) that complementarily binds to mRNA of fog2 or ush gene. Also, preferably, the material changing the activity of FOG2 or USH protein may be an activity inhibitor of FOG2 or USH protein, and the activity inhibitor of FOG2 or USH protein may be any one selected from the group induing materials to be complementarily bound to FOG2 or USH protein such as cytotoxic compound, peptide, peptide mimetics and antibody, but the present invention it not limited thereto.

The siRNA includes a 15- to 30-mer sense sequence selected from base sequences of RNA of gene (SERE ID:11 or SEQ ID:38) coding FOG2 or USH, and an antisense sequence complementarily bound to the sense sequence. Herein, the sense sequence preferably includes 19 to 27 bases, and FOG2 protein expression inhibitor more preferably has a base sequence represented by SEQ ID:119, although not particularly limited thereto.

Hereinafter, the present invention will be described in detail with reference to following Examples.

However, the following Examples are only for illustrative purposes and are not intended to limit the scope of the invention.

### Example 1. transgenic flies and culture of flies

The characteristics, suppliers, and literatures of transgenic flies used in the present invention are noted in Table 1. The flies were cultured in a standard drosophila medium, at 25°C before being used in experiments.

**[table 1]**

| drosophila | characteristic | supplier | reference |
|---|---|---|---|
| (mir-8△²) | miR-8 null flies | Steve Cohen | (Karres et al., 2007 |
| mir-8 gal4 | When combined with UAS-GFP, it expresses GFP under control of mir-8 enhancer | Kyoto Stock Centre | Karres et al., 2007 |
| ush1513 | It includes ush1513 hypomorph expressing a reduced level of ush as the result of a mutation in the promoter region | Pat Simpson | Cubadda, Yet al., 1997 |
| Cg gal4 | It expresses Gal4 in Fat body and anterior lymph gland | Young Joon Kim | Takata et al.,2004 |
| ppl gal4 | It expresses Gal4 specifically in fat body, which is reduced in salivary gland | M. Pankratz | Zinke et al., 1999 |

### Example 2. culture of cell lines

Cell lines noted in Table 2, bought from American Type Culture Collection (ATCC), were cultured at 374°C, under the condition of 5% CO₂.

The cultured cell lines were detached from a 75-cell culture flask by being treated with trypsin-EDTA (Trypsin-ethylenediamine tetraacetic acid, Invitrogen, US), added with serum containing medium so as to inactivate trypsin, and was subjected to precipitation through centrifugation. Supernatant was removed, and then culture medium according to each cell line was added to suspend cells. Live cells were stained with trypan blue dye exclusion test, counted by a hemocytometer, and subcultured in a 100 mm dish (5×10⁵ cells /flask).

**[table 2]**

| Cell line | medium |
|---|---|
| HepG2 | RPMI-1640 |
| Huh7 | |
| AsPC1 | |
| PANC1 | |
| SW480 | |
| MCF7 | |
| MDA MB 231 | |
| U2OS | |
| HeLa | |
| Hep3B | IMDM |
| HCT116 | |
| HCT116p53KD | |

| | |
|---|---|
| * IMDM(Iscove's Modified Dulbecco's Medium, Gibco, US): containing 10% FBS(Gibco) * RPMI-1640(Gibco): containing 10% FBS | |

### Example 3. small body size phenotype of mir-8 mutant flies

It has been known that when human miRNAs, that is, miR-200 family (miR-200a, miR-200b, miR-200c, miR-141, and miR-429) are transduced into human cell lines, cell growth is promoted (Park, SY et al., 2009, Nat Struct Mol Biol 16, 23-29). In MCF-7 cell lines not treated with anything, and MCF-7 cell lines transduced with siRNA targeting GFP (siGFP, SEQ ID:1: 5'-UGAAUUAGAUGGCGAUGUU-3', Samchonri), miR-200 family [miR-141(has-miR-141, SEQ ID:2: 5'-UAACACUGUCUGGUAAAGAUGG-3'), miR-200b(has-miR-200b, SEQ ID:3: 5'-UAAUACUGCCUGGUAAUGAUGA-3'), miR-429(has-miR-429, SEQ ID:. 4: 5'-UAAUACUGUCUGGUAAAACCGU-3')] and miR-29b (known to inhibit cell proliferation), on day 1, 4, 5, 6, 7 and 8, the inventors counted the number of cells and measured cell proliferation. As a result, they observed that cells treated with miR-200 family showed an increased cell proliferation as compared to cells not treated with anything, or cells treated witch siGFP, and that in cells treated with miR-29b, cell proliferation almost stopped from the 5^{th} day, thus, it was found again that miR-200 family promotes cell proliferation in human cells (see FIG. 6).

The miR-200 family is upregulated in certain cancer cells, including uterine cancer (lorio, MV et al., 2007, Cancer Res 67, 8699-8707; Nam, EJet al., 2008, Clin Cancer Res 14, 2690-2695), and is highly conserved in bilaterian animals, with miR-8 being the sole homolog of miR-200 in Drosophila mela-nogaster (Ibanez-Ventoso et al., 2008). Thus, the inventors used drosophila in order to discover the biological function of the miR-200. First, the inventors observed that the phenotype of the miR-8 null flies [mir-8△² (Karres, JS et al., 2007, Cell 131, 136-145)] shows a significantly small body size and body weight as compared to that of wild-type flies (see FIGs. 1 and 2). Groups of 5 flies (aged 3 to 5 days old after eclosion) were weighed. All flies were ice-anesthetized before weight measurement. Also, it is known that miR-8 null flies result in increased apoptosis in the brain and frequent occurrence of mal-formed legs and wings (Karres et al., 2007).

The determination of the final body size in insects during the larval stage is analogous to that which occurs during the human juvenile period (Edgar, 2006; Mirth and Riddiford, 2007). It is generally known that reduced body size in insects is caused by either slow larval growth, precocious early pupariation (pupal formation) that shortens the larval growth period, or both (Colombani et al., 2003; Edgar, 2006; Mirth and Riddiford, 2007). The inventors observed that at 100 hr after eclosion, miR-8 null larvae exhibit a significantly smaller body volume than do wild-type larvae (see FIG. 3). In each group, at 5hr after laying eggs, eggs were collected, and then number of new pupae and adults were counted every 12 hr. As a result, it was found that the onset of pupariation in miR-8 null flies was not significantly different from that in wild-type flies (see FIG. 3), and adult emergence (ecdysis of a chrysalis into an adult) was slightly delayed (about 12 hr) (see FIG. 3). Thus, the smaller body size of miR-8 null flies is likely to be caused by slower growth during the larval period rather than by precocious pupariation.

Insufficient food intake has been reported to accompany either precocious or delayed pupariation, depending on the onset of reduced feeding (Layalle, S et al., 2008, Dev Cell 15, 568-577; Mirth et al., 2005). However, the levels of Drosophila insulin-like peptides (Dilps), which are known to be reduced in starvation conditions (Calombani et al., 2003; Ikeya et al., 2002), were not downregulated in miR-8 null larvae. Given the unaffected onset time of pupariation and the levels of Dilps in miR-8 null larvae, the small body size of miR-8 null flies is unlikely due to reduced feeding.

Accordingly, in order to investigate whether the small body phenotype was caused by a reduction in cell size, cell number or both, the inventors collected 8 to 10 wings of miR-8 null flies and wild-type flies, and photographed them. Then, they counted the number of wing cilia with respect to the total of wing pixels, and calculated the relative size of a cell. Then, they divided the whole wing pixel area by a cell pixel area, and measured the relative size of a cell. As a result, wing cell number was reduced in the wing in miR-8 null flies as compared to that of wild-type flies, whereas wing cell size was not significantly different from that of wild-type flies (see FIG. 4). From this result, it was found that the reduced growth in the peripheral tissues of the miR-8 null flies was ascribed to decreased cell number rather than reduced cell size.

### Example 4. Determination of reduction of insulin signaling in miR-8 null flies

In order to understand why miR-8 null flies grow slowly, the inventors investigated the activities and gene expression of the proteins involved in insulin signaling in miR-8 null flies. First, by using an anti-phosphorylation Akt specific antibody, the level of activated Akt (p-Akt) was determined by western blotting (see FIG. 5). Specifically, from wild-type and miR-8 null flies, the same number of male and female flies were homogenized using 300 µℓ of lysis buffer (1% Triton X-100, 50 mM Tris pH7.4, 500 mM NaCl, 7.5 mM MgCl2, 0.2 mM EDTA, 1 mM NaVO4, 50 mM β-glycerophosphate, 1 mM DTT, and 25% glycerol) according to a known method (Lee, SB et al., 2007, EMBO Rep 8, 360-365), and then each group's protein extracted through RIPA buffer (25 mM Tris, pH 7.4, 150 mM NaCl, 1% NP-40, 1% sodium deoxycholate, and 0.1 % SDS) was separated by 6~10% SDS-PAGE. The separated protein was transferred to PVDF membrane through semi-dry transfer, blocked through culture in 2% BSA solution, and cultured in 2% BSA solution containing anti-phosphorylation-Akfi (P-Akt) antibody [1:1,000,(#9271 S, Ser473) Cell Signaling Technology, US]. Then, the protein was cultured in 2% BSA solution containing anti-mouse secondary antibody (SantaCruz, US) conjugated with Peroxidase, and developed on an X-ray film by using Chemiluminescent substrate (Roche, US) through immunochemistry before determination of a band. As a result, it was found that in miR-8 null flies, the p-Akt level was reduced, suggesting that Akt signaling is impaired in the absence of miR-8 (see FIG. 5).

Activated p-Akt is known to deactivate FOXO via phosphorylation. Phosphorylation of FOXO prevents nuclear localization of FOXO, which, in turn, results in the reduction of transcription of FOXO target genes. Thus, the inventors examined the expression level of 4EBP (the FOXO target gene) through Q-PCR of 4EBP gene in miR-8 null larvae. First, from third-stage larvae (at 96 hr after eclosion) of wild-type and miR-8 null flies, total RNA was extracted by using Trizol reagent. 1 µg of total RNA and 1 µℓ of Oligo-dT (Invitrogen, US) were charged with up to 50 µℓ of distilled water, and placed into SuperScript First-Strand Synthesis System (Invitrogen, US), followed by RT-PCR reaction. Through the reaction at 65°C 6 min, at 4°C 5 min, at 42°C for 60 min, at 95°C for 5 min, and at 4°C for 5 min, cDNA was synthesized. By using the cDNA as template, and using a primer pair of 4ebp sense primer (SEQ ID:5: 5'-ATGCAGCAACTGCCAAATC-3') and 4ebp antisense primer (SEQ ID:6: 5'-CCGAGAGAACAAACAAGGTGG--3'), and ABI SYBR PCR master mix (Applied Biosystems, US), in ABI Prism 7900 Sequence Detection System (Applied Biosystems, US), quantitative PCR on 4ebp mRNA was performed. Herein, by using a primer pair of rp49 sense primer (SEQ lD:7: 5'-AGGGTATCGACAACAGAGTG-3') and rp49 antisense primer (SEQ ID-8- 5'-CACCAGGAACTTCTTGAATC-3'), rp49 gene, as a quantitative control, was subjected to PCR, The expression level of 4ebp mRNA was converted into a relative value with respect to the level of rp49 mRNA, and a Ct (comparative cycle threshold) value was analyzed in accordance with a manual (User Bulletin 2, Applied Biosystems, US). The experiment was repeated three times, and the average value was (see FIG. z) indicated on a graph. From this result, it was found that in miR-8 null flies, insulin signaling was considerably reduced.

### Example 5. Determination of miR-8 expression level in drosophila tissues

The expression of miR-8 in the brain, wing discs, and leg discs of drosophila was observed by Cohen. et al. (Karres et al._{;} 2007), The inventors examined the spatial expression pattern of miR-8 in larvae, in the fat body, cuticle and brain tissues of the third-stage larvae (at 96 hr after egg-laying) of mir-8 enhancer trap GAL4 line drosophila (mir-8 gal4) (Karres et al., 2007), through immuno-staining according to a known method (Lee, Yet al., 2005, Nat Genet37, 305-310). As a result, the fat body showed the highest expression level of GFP (see FIG. 7). Also, from the total larva, fat body, gut, brain and cuticle, the total RNA was separated, and then miR-8 expression level was determined through Northen blotting. Specifically, from the fat body, gut, brain and cuticle, obtained from the whole of miR-8 Gal4 flies and through dissection of miR-8 Gal4 flies, total RNA was extracted by using Trizol reagent (Invitrogen, US), and 5.4 µg of total RNA of each kind of tissues was developed in 12.5% urea-polyacrylamide gel. The developed RNA was transferred to Zeta probe GT membrane (Biorad, US) via the flow of current. Oligonucleotide corresponding to miR-8 was end-labeled with [γ-³²ATP] and used as a probe for Northen blotting. The membrane treated with the probe was exposed to a phosphor imaging plate (fugi film, Japan), and was analyzed by a BAS-2500 system (Fugi). As a result, the larval fat body showed the highest miR-8 expression level (see FIG. 8).

### Example 6. Determination of increase in drosophila body size by fat body-specific expression of miR-8

Recent studies suggested that Drosophila fat body is an important organ in the control of energy metabolism and growth (Colombani et al., 2005; Colombani et al., 2003; Edgar, 2006; Leopold and Perrimon, 2007). Based on the assumption that the miR-8 expression level of larval fat body is important in control of drosophila body size, the inventors carried out the following experiments. They observed body weight of flies when miR-8 was expressed specifically in fat body of miR-8 null flies.

### <6-1> Production of amiR-8 and miR-200 miRNA expression vectors

The inventors performed cloning as described below in order to produce transgenic flies expressing miR-8 miRNA and miR-200c miRNA cluster. First, in order to obtain "a genome site of miR-8 miRNA" represented by SEQ ID-9(5'-TAATACTGTCAGGTAAAGATGTC-3'), genome DNA was extracted from flies by using Trizol reagent. Then, by using the genome DNA (3~5 µg) as a template, and using a primer pair of SEQ ID:10 (5'-TCACAAAGAATTCCTTATCGCCA-3') and SEQ ID:11(5'-GCTCTAGAATTTTCAGCCGCTTGTCTTCGC-3'), and PCR Mastermix (Promega, US), PCR was performed. After the reaction was completed, the PCR product of miR-8 was cleaved by restriction enzyme of EcoRI, and was cloned into pUAS vector cleaved by the same restriction enzyme so as to produce UAS-mir-8 vector.

Also, the inventors performed cloning as described below in order to produce transgenic flies expressing human miR-200c cluster (including miR-200c and mir-141). First, from HeLa cell lines expressing "miR-200c cluster" represented by SEQ ID-12, genome DNA was extracted by using Trizol reagent. Then, by using the genome DNA (3~5 µg) as a template, and using a primer pair of SEQ ID-13 (5'-CGGAATTCACGGTCGAGGGGCTTCGGAG-3') and SEQ ID-14 (5'-GCTCTAGACATGCTCCCAAGGCGGGAAGAC-3'), and PCR Mastermix (Promega, US), PCR product, on polynucleotide coding human miR-200c cluster (including miR-200c and mir-141), was obtained. The PCR product was cleaved by EcoRI and Xbal restriction enzymes, and was cloned into pUAS vector cleaved by the same restriction enzymes so as to produce UAS-human miR-200c cluster vector.

### <6-2> Production of miR-8 deficient flies expressing miR-8 specifically in fat body

mir-8△² and Cg gal4 noted in Table 1 were mated with each other, and then miR-8-deficient Cg gal4 flies, that is, mir8 CgG4 flies were selected. Also, mir-8△² and ppl gal4 noted in Table 1 were mated with each other, and then miR-8-deficient ppl gal4 flies, that is, mir8 pplG4 flies were selected. mir-8△ ², Cg gal4, and ppl gal4 flies, respectively, were mated with double balancer flies, and then through a standard mating method, mir8 CgG4 and mir8 pplG4 flies were obtained. Then, UAS-mir-8 vector produced in Example 1-1 was transfected into mir8 CgG4 and mir8 pplG4 through a known method, P-element-mediated germ line transformation, so as to obtain mir8 CgG4>mir8 and mir8 ppIG4>mir8 flies (Rubin GM et al., 1982, 357 Science 218: 348-353). Further, UAS-human miR-200c cluster vector produced in Example 1-1 was transfected into mir8 CgG4 and mir8 pplG4 in the same manner as described above so as to obtain mir8 CgG4>200c and mir8 ppIG4>200c flies.

### <6-3> Determination of the effect on drosophila growth by fat body-specific expression of miR-8 in miR-8 deficient flies

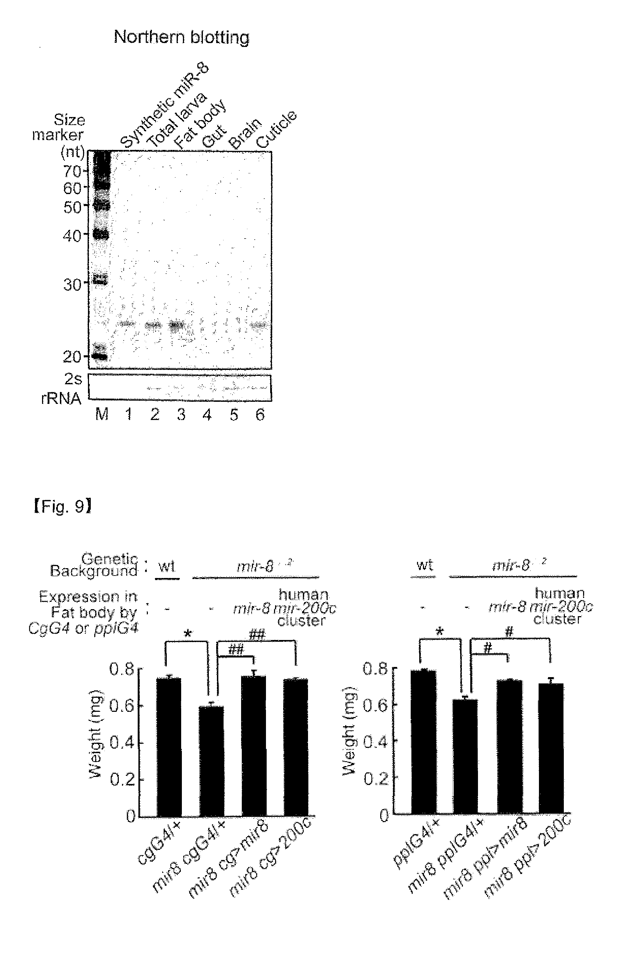

The inventors measured the body weight of Cg gal4, mir8 CgG4, mir8 CgG4>mir8 and mir8 CgG4>200c, or ppl gal4, mir8 pplG4, mir8 ppIG4>mir8 and mir8 ppIG4>200c, having fat body-specific Gal4 drivers, Cg and ppl, in the same manner as described in Example 3. As a result, when in miR-8 null flies, miR-8 was expressed specifically in fat body (mir8 CgG4>mir8 and mir8 pplG4), an increase in the body weight was similar to that of wild-type flies (Cg gal4 and ppl gal4) (see FIG. 9). This supports that miR-8 functions specifically in fat body, and suggests that miR-8 in fat body is important the whole body growth. Also, when human miR-200 cluster was expressed in drosophila fat body (mir8 CgG4>mir8 and mir8 ppIG4>mir8), the body weight of flies was increased (see FIG. 9). Human miR-200 family miRNAs, which are located in two chromosomal clusters, have extensive homology to miR-8 (see FIG. 11). The fact that human miR-200c clusters effectively replace the role of miR-8 suggests that these human miRNAs can be processed by the drosophila miRNA processing machinery, and that they share a conserved biological function.

### <6-4> Determination of the effect on drosophila growth by neuronal cell-specific expression of miR-8 in miR-8 deficient flies

As mentioned above, miR-8 is known to be expressed in the central nervous system, and to prevent neurodegeneration (Karres et al., 2007). In order to investigate whether miR-8 in neuronal cells participates in the regulation of organismal growth by indirectly regulating feeding behavior, mir-8△² and elav gal4 noted in Table 1 were mated with each other, and then miR-8-deficient elav gal4 flies, that is, mir8 elavG4 flies, were selected. The mir-8△² and elav gal4 flies, respectively, were mated with double balancer flies, and then through a standard mating method, flies including both mir-8△² and elav gal4 were obtained. UAS-mir-8 vector produced in Example 1-1 was transfected into mir8 elavG4 in the same manner as described above so as to obtain mir8 elavG4>mir8 flies. The body weight of mir8 elavG4 and mir8 elavG4>mir8 was measured. As a result, the body weights of flies were similar irrespective of expression of miR-8 in neuronal cells (see FIG. 10). This indicates that the function of miR-8 in the fat body is spatio-temporally specifically different from neuronal cells.

### Example 7. Identification of target genes of miR-8 and miR-200

To further understand the molecular function of miR-8, the inventors set out to identify miR-8 target genes responsible for body size control. Because miR-8 is a highly conserved miRNA and because the human homologs of miR-8 promote cell proliferation in human cells (see FIG. 12), the inventors assumed that the target genes of miR-8 are related to cell proliferation phenotype. The inventors listed target gene candidates of both fly miR-8 and human mi-200 miRNAs by using five different target prediction programs (see FIG. 14): miRanda(John et al., 2004), miTarget(Kim et al., 2006), microT(Kiriakidouet al., 2004), PicTar(Krek et al., 2005), and Target Scan(Lewis et al., 2005). The listed candidates were divided into two groups of fly genes and human genes, and then the groups were compared with each other to identify homologous gene pairs through Ensembl Orthologous data(//www.ensembJ-org/), NCBI Homologene data(//www.ncbi.nlm.nih.gov/Homologene/) and Inparanoid data(//inparanoid.sbc.su.se/) (see FIG. 13). From the target candidate list, the inventors selected, as target genes, drosophila genes ush, Lap1, CG8445, dbo, Lar, Ced-12, CG12333, cbt, Scr, pan, pnut, Spri, Shc, Rassf and CG5 608, that are known as tumor suppressors or negative regulators of cell proliferation in at least one species, and the respective human orthologs of the drosophila genes, FOG2, SEPT7, RIN2, SHC1, HOXB5, KLF11, TCF7L1, ERBB21P, ELMO2, BAP1, W DR37, KLHL20, PTPRD, RASSF2 and VAC14. The Drosophila gene u-shaped (ush) and its human homolog Friend of GATA 2 (FOG2, also known as ZFPM2) were the most frequently predicted gene pair conserved in both species.

### Example 8. Identification of miR-8 target genes performing an important role in regulation of body growth

### <8-1> Production of reporter gene expression vectors for target genes of miR-8 and/or miR-200 miRNAs

In order to determined whether the target genes identified in Example 7 are in actuality targets of miR-8 and miR-200 family miRNAs, the inventors performed cloning. In other words, they inserted a luciferase gene into downstream of each target gene's 3'UTR containing the miRNA target sites. Specifically, in order to obtain cDNAs of respective target genes, from drosophila S2 cell lines or human Hela cell lines, total RNA was extracted by using RNeasy mini kit (QIAGEN, US). 1 µg of total RNA and 1 µℓ of Oligo-dT (Invitrogen, US) were charged with up to 50 µℓ of distilled water, and placed into SuperScript First-Strand Synthesis System (Invitrogen, US), followed by RT-PCR reaction. Through the reaction at 65°C for 6 min, at 4°C for 5 min, at 42°C for 60 min, at 95°C for 5 min, and at 4°C for 5 min, cDNA was synthesized. By using the cDNA (3~5 µg) as template, and using a primer pair on respective genes noted in Table 3 and PCR Mastermix (Promega, US), PCR was performed on 3'UTR of ush, Lap1, CG8445, dbo, Lar, Ced-12, CG12333, cbt, FOG2, SEPT7, RIN2, SHC1, HOXB5, LF11, TCF7L1, ERBB2IP, ELMO2, BAP1, WDR37, KLHL20, PTPRD, RASSF2 and VAC14 from among the target genes selected in Example 7. Herein, PCRP on atro as a drosophila control gene was also performed. After the reaction was completed, the PCR product on each gene's 3'UTR region was cleaved by each of the following restriction enzymes:
wherein for ush, atro, CG8445, cbt, lap1, Ced-12, CG12333, dbo, and lar, Sacl and BamHI restriction enzymes were used;
for FOG2, SEPT7, HOXB5, KLF11, ERBB21P, ELMO2, BAP1, and PTPRD, Xbal and EcoRI restriction enzymes were used;
for RIN2, SHC1, WDR37, KLHL20, RASSF2, and VAC14, Xbal restriction enzyme was used; and
for TCF7I1, EcoRI and EcoRV restriction enzymes were used.

Then, it was cloned into pAC5.1 luciferase vector (a kind gift of Elisa Izaurralde) cleaved by the same restriction enzymes so as to produce a reporter plasmid having a miR-8 or miR-200 family target sequence in luciferase gene's 3'UTR sequence. These results are noted in Table 4.

**[table 3]**

| derived | gene | SEQ ID: | primer sequence | | | |
|---|---|---|---|---|---|---|
| | | | SEQ ID: | sense primer | SEQ ID: | antisense primer |
| Drosophila | ush | SEQ ID:15 | SEQ ID:16 | 5'-ATCGATGAG CTCGAAAGCCA GCTGCGGTGG GAG-3' | SEQ ID:17 | 5'-ACGCGGATC CGGCAGCACTC AAATCGTTCGTT G-3' |
| Drosophila | atro | SEQ ID:18 | SEQ ID:19 | 5'-ATCGATGAG CTCGTGGACA GACAGCAATTG TAGAGAGC-3' | SEQ ID:20 | 5'-ACGCGGATC CGCAGAGAAGG TTTTCTGGGATT TGG-3' |
| Drosophila | CG8445 | SEQ ID:21 | SEQ ID-22 | 5'-ATCGATGAG CTCCCAGACGA CCAGCATGTAC | SEQ ID-23 | 5'-ACGCGGATC CCAATAACGTTA GGATTCACCCG |
| | | | | ATG-3' | | ATT-3' |
| Drosophila | cbt | SEQ ID:24 | SEQ ID:25 | 5'-ATCGATGAG CTCGAAGAGCA ATGCGAGCAG CATC-3' | SEQ ID:26 | 5'-ACGCGGATC CGATTGGAATC GTGCGATGATC C-3' |
| Drosophila | Lapl | SEQ ID:27 | SEQ ID:28 | 5'-GAGCTCTTAT TGTCCAATCGA TCGAAGTGTC- 3' | SEQ ID:29 | 5'-GGATCCGCG TGTGTTCTTCAA TAATGCA-3' |
| Drosophila | Med-12 | SEQ ID:30 | SEQ ID:31 | 5'-GAGCTCCAT AACGAGCACAA TTACTTCACG-3' | SEQ ID:32 | 5'-GGATCCCGTC ACCAGTTTGTTC ATTTTG-3' |
| Drosophila | CG12 333 | SEQ ID:33 | SEQ ID:34 | 5'-GAGCTCTCT GCGAAATCCCA AACATCT-3' | SEQ ID:35 | 5'-GGATCCCGCT ATGCCACGACTA TTTGG-3' |
| Drosophila | dbo | SEQ ID:36 | SEQ ID:37 | 5'-GAGCTCTGT TTATTTGGTAGA GCCTAAAGCG- 3' | SEQ ID:38 | 5'-GGATCCCCAC CGGCAACATTTA GATAT-3' |
| Drosophila | Lar | SEQ ID:39 | SEQ ID-.40 | 5'-GAGCTCCAA TGGGATTGCCA GGTCCAC-3' | SEQ ID:41 | 5'-GGATCCTCCC CCAAAGAATCGT AATGG-3' |
| human | FOG2 | SEQ ID:42 | SEQ ID:43 | 5'-GCTCTAGAC AGTCACCTTTG GTATCAGTGTT TAG-3' | SEQ ID:44 | 5'-CGGAATTCCA TACTTGCAGCAT TGAGTTTAGGG- 3' |
| human | SEPT7 | SEQ ID:45 | SEQ ID:46 | 5'-GCTCTAGAG ACCACCAGTTA ACGTATTAGTT GCC-3' | SEQ ID:47 | 5'-CGGAATTCGA GGCCATGATTC CCATTTCTAGT-3 ' |
| human | RIN2 | SEQ ID:48 | SEQ ID:49 | 5'-GCTCTAGAC TTCCCAGTGGT GCATCCAAAGG -3' | SEQ ID-50 | 5'-CATAAGTTTAA TTTCACTGCAG GTTCTG-3' |
| human | SHC1 | SEQ ID:51 | SEQ ID:52 | 5'-GCTCTAGAC TAGCGCTCTCT TCCAGAAGATG C-3' | SEQ ID:53 | 5'-GTACTCAGGA ACTGCAGGTTTT TGC-3' |
| human | HOX B5 | SEQ ID:54 | SECT ID:55 | 5'-GCTCTAGAC AGAGGAGCCC AGCGGCCCA-3' | SE Q ID:56 | 5'-CGGAATTCCA CTGAGACAGGC CCCGCAAAGAC A-3' |
| human | KLF11 | SEQ ID:57 | SEQ ID:58 | 5'-TCTAGAGGT CCATTAGGACA | SEQ ID:59 | 5'-GAATTCCTGC CCAGGCAATAA |
| | | | | TCACTC-3' | | AGTG-3' |
| human | TCF7L1 | SEQ ID:60 | SEQ ID:61 | 5'-GAATTCGAC CCCTGCAGGC TGTCAC-3' | SEQ ID:62 | 5'-GATATCGGGA GTTTAAATTGCT CC-3' |
| human | ERBB 2lP | SEQ ID-63 | SEQ ID:64 | 5'-GGGGAGAGC TACGTGGTAGT ATG | SEQ ID:65 | 5'-CCAATACTTT CAAAGAAAACTT -3' |
| human | ELMO2 | SEQ ID:66 | SEQ ID:67 | 5'-CTCTAGAAG ACCCCAGGAA GTTGGCCTT-3' | SEQ ID-68 | 5'-GAATTCCCCC AAGGTTCCATC CTAAGA-3' |
| human | BAP1 | SEQ ID:69 | SEQ ID:70 | 5'-CTCTAGACC TGACTCTGCAG CCCACTCTT-3' | SEQ ID:71 | 5'-GAATTCAGGA AGAGCTGAGTG GTGCCAG-3' |
| human | WDR37 | SEQ ID:72 | SEQ ID:73 | 5'-TCTAGAGTCT GGGTGTCTTAG GATGATGG-3' | SEQ ID-74 | 5'-TCTAGAGTTC AATTTCTAGCTC ATCGTGATG-3' |
| human | KLHL20 | SEQ ID-75 | SEQ ID:76 | 5'-TCTAGAAGA AAAATCTTTGAA TAAGACTAAC-3' | SEQ ID:77 | 5'-TCTAGAAGCT AAAATTTTTTTTAA TGGTAA-3' |
| human | PTPRD | SEQ ID:78 | SEQ ID:79 | 5'-CTCTAGACG TTATGTGGCTT TGCCATCC-3' | SEQ ID:80 | 5'-GAATTCGTAA CAAATGGAACTT GTGTCAGC-3' |
| human | RASSF2 | SEQ ID:81 | SEQ ID:82 | 5'-TCTAGATGTG TGCACACATGG TACGTGTC-3' | SEQ ID:83 | 5'-TCTAGATGTAT GGCTCCCTTGC TGAGG-3' |
| human | VAC14 | SEQ ID:84 | SEQ ID:85 | 5'-TCTAGAAACA CTAAGGGTCGT CACGCC-3' | SEQ ID:86 | 5'-TCTAGACTTT GTGCTGGTGCC TACGTG-3' |

**[Table 4]**

| | gene | GenBank access no | cloning site of 3'UTR | Vector name |
|---|---|---|---|---|
| Drosophila gene | ush | NM_057432.2 | 4154-4168 | pAC-ush |
| | atro | NM_206303.1 | 6524-8298 | pAC-atro |
| | CG8445 | NM_176194.1 | 1718-2091 | pAC-CG8445 |
| | cbt | NM_164384.1 | 1495-2003 | pAC-cbt |
| | Lap1 | NM_137163.3 | 3817-4029 | pAC-Lap1 |
| | Ced-12 | NM_135704.2 | 2285-2451 | pAC-Ced-12 |
| | CG12333 | NM_142466.2 | 1761-1948 | pAC-CG12333 |
| | dbo | NM_080250.2 | 2416-2604 | pAC-dbo |
| | Lar | NM_078880.2 | 6218-6458 | pAC-Lar |
| Human gene | FOG2 | NM_012082.2 | 3494-4322 | pAC-FOG2 |
| | SEPT7 | NM_001788.4 | 1518-2410 | pAC-SEPT7 |
| | RIN2 | NM_018993.2 | 2738-3963 | pAC-RIN2 |
| | SHC1 | NM_003029.3 | 1552-2860 | pAC-SHC1 |
| | HOXB5 | NM_002147.3 | 877-1759 | pAC-H OXBS |
| | KLF11 | NN_003597.4 | 1704-3867 | pAC-KLF11 |
| | TCF7L1 | NM_031283.1 | 1851-2717 | pAC-TCF7L1 |
| | ERBB2IP | NM_018695.2 | 5022-6826 | pAC-ERBB21P |
| | ELMO2 | NM_133171.3 | 3235-3554 | pAC-ELM02 |
| | BAP1 | NM_004656.2 | 2317-3244 | pAC-BAP1 |
| | WDR37 | NM_014023.3 | 3627-4589 | pAC-WDR37 |
| | KLHL20 | NM_014458.3 | 3229-3462 | pAC-KLHL20 |
| | PTPRD | NM_002839.2 | 9771-10044 | pAC-PTPRD |
| | RASSF2 | NM_014737.2 | 1436-5354 | pAC-RASSF2 |
| | VAC14 | NM_018052.3 | 2649-3040 | pAC-VAC14 |

### <8-2> Determination of regulation on a target gene by miR-8 and/or miR-200 miRNAs

Reporter plasmids and renilla luciferase expression vectors (Promega, US), as noted in Table 4, and miRNAs of miR-200 family of miR-141, miR-200a (hsa-miR-200a, SEQ ID:87: 5'-UAACACUGUCUGGUAACGAUGU-3'), miR-200b, miR-200c (has-miR-200c, SEQ ID:88: 5'-UAAUACUGCCGGGUAAUGAUGGA-3') and miR-429, and miR-8 (dme-miR-8, SEQ ID: 89: 5'-UAAUACUGUCAGGUAAAGAUGUC-3') were bought from Samchonri and cotransfected into drosophila S2 cell lines or human Hela cell lines. Then, the activities of luciferase were measured through dual-luciferase assay (Promega, US) by a luminometer (Biorad). The results are noted in Table 5. Then, 7 gene pairs that respond both to miR-8 and to mir-200 family miRNAs in Drosophila and human cells, respectively were identified (see FIG. 15).

**[Table 5]**

| Fly gene | Programs predicting this gene | Repression fold miR-8 | Human ortholo gue | Programs predicting this gene | Repression fold | | | | |
|---|---|---|---|---|---|---|---|---|---|
| | | | | | mi R-141 | mi R-200a | mi R-200b | mi R-200c | mi R-429 |
| *ush* | ts.pt.mr. mt | **0.71** | *FOG2* | ts.pt.mr. mc | **0.59** | **0.64** | **0.37** | **0.32** | **0.47** |
| *Lap1* | ts.pt.mr. mt | **0.46** | *ERBB 2IP* | mr.mt | **0.7** | **1.19** | **0.57** | **0.59** | **0.54** |
| *CG 8445* | ts.mr | **0.43** | *BAPI* | ts.pt.mr. mt | 0.84 | 1.04 | **0.79** | **0.74** | **0.67** |
| *dbo* | mt | **0.54** | *KLHL 20* | mt | 0.84 | 1.02 | 0.85 | **0.69** | **0.55** |
| *Lar* | ts.pt.mr. mt | **0.42** | *PTPR D* | ts.mr | 0.89 | 1.25 | 0.89 | 0.97 | **0.75** |
| *Ced-12* | ts.pt.mt | **0.51** | *ELMO 2* | mr.mt | **0.73** | 1.22 | 9.04 | 0.86 | 0.89 |
| *CG 92 333* | ts | **0.59** | *WAR 37* | mr | 1.07 | **0.76** | 1.16 | 1.21 | 1.31 |
| *cbt* | mt | **0.75** | *KLF11* | mt | 1.03 | 1.20 | 1.02 | 1.07 | 1.07 |
| *Scr* | ts.pt.mr. mt | n.d. | *HOXB 5* | ts.pt | 0.92 | 1.16 | **0.73** | 0.87 | **0.72** |
| *pan* | mt | n.d. | *TCF7L1* | mt | 0.91 | 1.17 | 0.81 | 0.80 | **0.71** |
| *pnut* | ts.pt.mr | n.d. | *SEPT7* | ts.pt.mr | 1.00 | 0.97 | 0.95 | 1.29 | 1.14 |
| *spri* | ts. pt.mr | n.d. | *RIN2* | mt | 1.10 | 2.49 | 1.91 | 1.30 | 1.41 |
| *Shc* | ts.pt | n.d. | *SHC1* | ts | 1.64 | 1.63 | 1.41 | 1.85 | 2.17 |
| *Rassf* | mt | n.d. | *RASSF2* | mt | 0.90 | 1.36 | 1.28 | 0.99 | 1.01 |
| *CG 5608* | mt | n.d. | *VAC14* | mr | 0.99 | 1.19 | 1.20 | 1.20 | 1.17 |

| | | | | | | | | | |
|---|---|---|---|---|---|---|---|---|---|
| 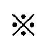 Ts: Targetscan; pt: Pictar;, mr: Miranda; mt: miTarget; mc: microT 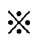 average of multiples of expression levels in at least twice independent experiments 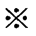 a value reduced to 0.8 times or more is bold-faced 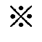 n.d.: not detected | | | | | | | | | |

### Example 9. ush, a target gene of miR-8 regulating body growth

### <9-1> Determination of phenotype encoded by miR-8 target gene

In order to determine which targets among target genes identified in Example 8-2 have activities physiologically relevant to the phenotype observed in miR-8 null flies, the following experiment was performed. Specifically, into larvae of mir8 CgG4 flies produced in Example 6-2, UAS vector (Vienna RNAi Library Centre) expressing dsRNA for target genes such as ush, Lap1, Ced-12, CG8445 and CG12333, and a control gene, atro (known to prevent neurodegeneration) was transfected in the same manner as described in Example 6-2. Then, transgenic flies of mir8 Cg>ush i, mir8 Cg>Lap1 i, mir8 Cg>Ced-12 i, mir8 Cg>CG8445 i, mir8 Cg>CG12333 i and mir8 Cg>atro i, in which ush, Lap1, Ced-12, CG8445, CG12333 and atro were knocked down specifically in fat body, were produced.

It was determined whether in the knockdown flies, each gene was in actuality knocked down, through quantitative -RT-PCR. Specifically, from third-stage larvae of the transgenic flies (at 96 hr after eclosion), total RNA was extracted. Then, by using a primer pair on each gene noted in Table 6, quantitative PCR on ush, Lap1, Ced-12, CG8445, CG12333 or atro mRNA was performed in the same manner as described in Example 4. Herein, by using a primer pair of rp49 in Example 4, rp49 gene, as a quantitative control, was subjected to PCR. The expression levels of ush, Lap1, Ced-12, CG8445, CG12333 and atro mRNAs were converted into relative values with respect to the level of rp49 mRNA, and a Ct (comparative cycle threshold) value was analyzed in accordance with a manual (User Bulletin 2, Applied Biosystems, US). The experiment was repeated three times, and the average value was indicated on a graph (see FIG. 17). From the result of quantitative -RT-PCR, it was determined that other genes, except for Laps, were knocked down (see FIG. 17).

**[table 6]**

| gene | primer | | | |
|---|---|---|---|---|
| | sense primer | | antisense primer | |
| ush | SEQ ID:90 | 5'-ATTAAATCGGAGCC TCTGGA-3' | SEQ ID:91 | 5'-GGCTGTCGCTGGC CT-3' |
| Ced-12 | SEQ ID:92 | 5'-GTGCCGGAAAACT CATTC-3' | SEQ ID:93 | 5'-GTACAGCTGGTGG GTCATCT-3' |
| CG8445 | SEQ ID:94 | 5'-CATGAATCACGGC GG-3' | SEQ ID:95 | 5'-TGAACTTATCGTAG TTGTGGGT-3' |
| CG12333 | SEQ ID:96 | 5'-GGACTTCCGAGAG GCAAT-3' | SEQ ID:97 | 5'-ATCGCAGTCCAGC AGC-3' |
| Lap1 | SEQ ID:98 | 5'-CACAACAAGTGCAA TATACGG-3' | SEQ ID:99 | 5'-TGCTATTCAAAGCA TCTTGGT-3' |
| atro | SEQ ID:100 | 5'-AGCAGGATACGCC CAAC-3' | SEQ ID:101 | 5'-TGCGGCTACCGGA CT-3' |

### <9-2> ush, target gene of miR-8 miRNA

Body weight measurement of CgG4 (wild-type) noted in Table 1, mir8 CgG4 (miR-8 null flies) and mir8 Cg>mir8 (miR-8 is expressed specifically in fat body in miR-8 null flies) produced in Example 6-2, and mir8 Cg>ush i, mir8 Cg>Lap1 i, mir8 Cg>Ced-12) i, mir8 Cg>CG8445 i, mir8 Cg>CG12333 i and mir8 Cg>atro i flies, produced in Example 9-1 was performed in the same manner as described in Example 3. As a result, when in miR-8 null flies, miR-8 was expressed specifically in fat body, an increase in the body weight was similar to that of wild-type flies. Also, when in miR-8 null flies, ush was knocked down specifically in fat body, the same effect was achieved (see FIG. 16). From this result, it was found that a target gene of miR-8 miRNA is ush.

Because a previous study showed that miR-8 targets atrophin (atro) to prevent neurodegeneration (Karres et al., 2007), the inventors tested whether atro is also involved in body size regulation by being expressed in fat body. It is known that atro is expressed along drosophila Atlas (first cervical vertebra) in larval fat body (Chintapalli, VR et al., 2007, Nat Genet 39, 715-720). Knockdown of atro in the fat body failed to rescue the small body phenotype of miR-8 null flies (see FIG. 16). From this result, it was found that the function of miR-8 in the prevention of neurodegeneration (Karres et al., 2007) may be separate from its function in body growth, not only spatially but also at the molecular level.

### <9-3> Determination of phenotype of ush knockdown flies

In order to exclude possible off-target effects of ush knockdown by dsRNA, the inventors produced ush1513 heterozygotes (w¹¹¹⁸ush1518) and miR-8 null ush1513 heterozygotes (mir-8^{□2} ush1518) by mating ush1513 flies (Cubadda, Yet al., 1997, Genes Dev 11, 3083-3095) with wild-type flies (w¹¹¹⁸) or miR-8 null flies (mir-8^{□2}). The mir-8^{□2} and ush1513 flies, respectively, were mated with double balancer flies. Then, a standard mating method was used to produce flies, in which each mutant was included in one individual. The body sizes of w¹¹¹⁸, w¹¹¹⁸ush1518, mir-8^{□2} and mir-8^{□2} ush1518 flies was measured in the same manner as described in Example 3, and compared to each other. As a result, ush1513 heterozygotes have larger adult bodies than do the wild-type flies. Further, when miR-8 null flies expressed a reduced level of ush, it was observed that the small body size phenotype was rescued (see FIG. 18). This result indicates that USH suppresses body growth.

### Example 10. Determination of suppression of USH expression by miR-8

### <10-1> Determination of expression level of ush mRNA in miR-8 null flies

In order to determine whether mRNA expression of USH (selected as a target gene of miR-8) was in actuality increased in miR-8 null flies, the inventors performed quantitative RT-PCR. On the total RNA separated from whole larva or larval fat body, quantitative RT-PCR was performed in the same manner as described in Example 4 so as to determine the endogenous ush mRNA level. Herein, a primer pair of ush sense primer (SEQ ID:102: 5'-CGAATTCCGATGTATCCAACG-3') and ush antisense primer (SEQ ID:103: 5'-GGAGTTGTTGTTCTCGTGCACC-3') was used. Also, by using a primer pair on rp49 in Example 4, rp49 as a quantitative control was subjected to PCR.

As a result, upregualtion of the ush mRNA in whole miR-8 null larva was less prominent (~1.3 fold) while the ush mRNA was significantly upregulated in the larval fat body (~2.0 fold). This suggests that miR-8 suppresses ush in the fat body (see FIG. 19). Thus, it was found that ush is more strongly suppressed in the fat body than in other body parts.

### <10-2> Determination of expression level of USH protein in miR-8 null flies

The inventors performed western blotting in order to determine whether the protein expression of USH (selected as a target gene of miR-8) was in actuality increased in miR-8 null flies.

Also, on whole larva or larval fat body, by using anti-USH antibody (Peptron, Korea), western blotting was performed in the same manner as described in Example 4. Herein, by using anti-ACTIN antibody, expression level of ACTIN, as a quantitative control, was also observed.

As a result, USH protein levels were more dramatically reduced than the mRNA levels (see FIG. 20), indicating that miR-8 represses USH expression by both mRNA destabilization and translational inhibition.

### <10-3> Determination of regulation on miR target site of ush 3'UTR by miR-8

### <10-3-1> Production of a vector having a mutation in ush 3'UTR miR target site

The inventors performed the following cloning in order to determine whether a mutation of the miR-8 target site in the 3'UTR of ush cause a change in the regulation by miR-8.

Specifically, the PCR product on 3'UTR region of ush, obtained through PCR in 8-1, was cleaved by BamH1 and Sac I restriction enzymes, respectively, and was cloned into pGL3_CMV vector (modified from Promega's pGL3, Kim et al., Nucleic Acid Research, 2009) cleaved by the same restriction enzymes so as to product a reporter plasmid having an miR-8 target sequence in 3'UTR sequence of luciferase, gene. The plasmid was called, "Luc-ush 3'UTR".

Also, by using a primer pair of SEQ ID: 104(5'-CAACCATCGACGACAACTCTCCCGGAAAATCTCC-3') and SEQ ID: 105(5'-GGAGATTTTCCGGGAGAGTTGTCGTCGATGG TTG-3'), and QuickChange Site-Directed Mutagenesis kit (Stratagene, US), a point mutation was introduced into the miR-8 target site in the 3'UTR of ush (Luc-ush 3'UTR). The mutant was called "Luc-ush 3'UTR mut".

Further, a genome site of miR-8 miRNA, obtained through PCR in Example 6-1, was cleaved by EcoRI and Xbal restriction enzymes, respectively, and was cloned into pAC 5.1 vector (Invitrogen) cleaved by the same restriction enzymes so as to produce "Acmir8" vector.

### <10-3-2> Determination of regulation on mutation in mutant miR target site in ush 3'UTR, by miR-8

Drosophila S2 cells were co-transfected with pGL3 CI111V vector (Luc null, positive control), or Luc-ush 3'UTR or Luc-ush 3'UTR mut produced in Example 10-3-1, together with Acmir8 and renilla luciferase expression vector (Rehwinkel J et al., RNA,2005) according to a known method (DDAB) (Han, 1995). Then, the activity of luciferase was measured by dual-luciferase assay (Promega, US) in a luminometer (Biorad). As a result, it was found that when the miR-8 target site in 3'UTR of luciferase was mutated, the luciferase activity was increased up to a level similar level to that of the positive control (see FIG. 21). This suggests that suppression of USH expression is mediated through direct binding of miR-8 to the predicted target site. Further, putative target sites for miR-8 were found in all Drosophilid species examined, including distant species such as D. virilis and D. grimshawi. Thus, it was found that ush is an authentic target of miR-8.

### Example 11. Determination of regulation on PI3K by miR-8 and USH

Several studies suggested that insulin signaling of larval fat body regulates organismal growth in a non-autonomous manner (Britton et al., 2002; Colombani et al., 2005). In order to confirm this finding, the inventors tested whether the suppression of insulin signaling in the larval fat body inhibits drosophila growth. Specifically, CgC4 flies in Table 1 were mated with PI3K DN flies (expressing dominant negative PI3K) or dsInR flies (expressing dsRNA for InR) so as to produce Cg>PI3K DN (expressing dominant negative PI3K specifically in fat body) and Cg>dsInR (expressing dsRNA for InR specifically in fat body). The body sizes of the CgC4, Cg>PI3K DN and Cg>dsInR flies were measured in the same manner as described in Example 3, and compared to each other. As a result, Cg>PI3K DN and Cg>dsInR flies have smaller adult bodies than do CgC4 flies. Also, wing cell sizes of CgC4 and Cg>PI3K DN flies were measured in the same manner as described in Example 3, and compared to each other. As a result, there was no significant difference between them. Also, when the wing cell numbers of CgC4 and Cg>dslnR flies were measured in the same manner as described in Example 3, and compared to each other. Cg>dsInR flies have fewer wing cells than CgC4 flies (see FIG. 18).

### Example 12. Determination of specific disruption of insulin signaling in fat body of miR-8 null flies

Based on the finding that when insulin signaling was inhibited in drosophila fat body, flies (see FIG. 22) having small body sizes and reduced wing cell numbers, like miR-8 null flies (see FIG. 4), were obtained, the inventors assumed that USH inhibits the body growth by suppressing insulin signaling. Then, they examined whether insulin signaling is defective in the fat body of miR-8 null flies. Specifically, tGPH vector (expressing a GFP protein fused to a PH domain, Britton et al., 2002) was transduced into wild-type larvae or miR-8 null larvae in the same manner as described in Example 6-2. Then, third-stage larvae of each group were dissected, and on fat body, immuno-staining was performed in the same manner as described in Example 5. Herein, for tGFP staining, Zamboni's fixative (4% paraformaldehyde, 7.5% saturated picric acid in PBS) was used for fixation, and also anti-GFP antibody (Sigma, US) and anti-FOXO antibody (obtained from 1:200, O. Puig) were used. The fat body tissue test sample of the wild-type larvae or the miR-8 null larvae was stained with phalloidin and DAPI, and then actin and nucleus were observed, respectively. The activity of PI3K can be measured by monitoring the membrane-anchored PIP3 through GFP signal expressed by tGFP because PH domains bind to the membrane-anchored PIP3 (membraneanchored phosphatidylinositol-3,4,5-triphosphate) produced by PI3K. The inventors found that PI3K activity was downregulated in fat bodies of miR-8 null flies (see FIG. 23), and that FOXO was more strongly localized to the nucleus (see FIG. 23), as compared to wild-type flies. Moreover, in fat body tissues of wild-type larvae or miR-8 null larvae, western blotting was performed, in the same manner as described in Example 4, by using anti-p-Akt(phosphorylation-Akt) antibody (Ser505, Cell Signaling), anti-Akt antibody (Cell Signaling, US), anti-p-JNK (phosphorytation-JNK) antibody (Santa cruz, US) and anti-JNK antibody (Santa cruz, US). As a result, as compared to the control, the level of p-Akt was significantly reduced in the fat body of miR-8 null flies, whereas the phosphorylated-JNK level did not change (see FIG. 24). From this result, it was found that insulin signaling was specifically disrupted in the fat bodies of miR-8 null larvae.

### Example 13. Promotion of cell growth by PI3K activation of miR-8 in a cell-autonomous manner

### <13-1> Determination of PI8K activation by miR-8

To more precisely analyze miR-8's function in fat cells, the inventors produced GAL4-overexpressing mosaic clones of miR-8 in the fat body of miR-8 heterozygote according to a known method (Seth S. Blair, Generating Imaginal Disc Clones in Drosophila, CSH Protocols, 2007; pdb.prot4793). The mosaic fat cells overexpressing miR-8 were transfected with tGPH vector in the same manner as described in Example 12, and then were subjected to immuno-staining using anti-GFP antibody (membrane staining, PI3K activity measurement) and anti-CD2 antibody (miR-8 null cell staining). As a result, in the membrane of cells overexpressing miR-8, the GFP signal was augmented. Also, the cell size was larger than that in miR-8 null cells (see FIG. 25). This result indicates that miR-8 promotes cell growth by activating PI3K in a cell-autonomous manner.

### <13-2> Determination of fat body tissue-specific cell-autonomous growth promotion by miR-8

in order to observe the loss of function phenotype through Twin-spot analysis (mitotic clone analysis), the inventors next generated mitotic null clones of miR-8 by using a FLP/FRT system (Golic, K.G.and Lindquist, S, 1989). Herein, when heat shock is applied, chromosomal recombination is specifically caused. Then, after cell division, the cells are divided into null clones and twin-spot cells with two phenotypes. Cells of the miR-8 null clone were smaller than the adjacent cells in the twin spot-the cells harboring wild-type copies of miR-8 (see FIGs. 26 and 27). This suggests that miR-8 promotes fat cell growth in a cell-autonomous manner, as expected if miR-8 enhances insulin signaling in the fat body. The inventors found that fewer (or no) null clone cells next to the twin spot cells when the mitotic clones were induced at embryonic stage or newly hatched larval stage. This suggests the frequent failure of proliferation and survival of miR-8 null cells during larval development (see FIG. 26). When twin-spot analysis was carried out in the wing or eye disc in the same manner as described above, null clones of miR-8 were generated but little growth defect in these organs was found (Karres et al., 2007)(see FIG. 28). From this result, it can be found that the effect of miR-8 on cell growth is dependent on tissue type, which may be explained by the fact that USH is present in the fat body but not in wing precursor cells or the eye disc (Cubadda, et al., 1997)(see FIG. 29).

### Example 14. Determination of the role of ush in an insulin signaling process

### <14-1> Determination of the role of USH in an insulin signaling process

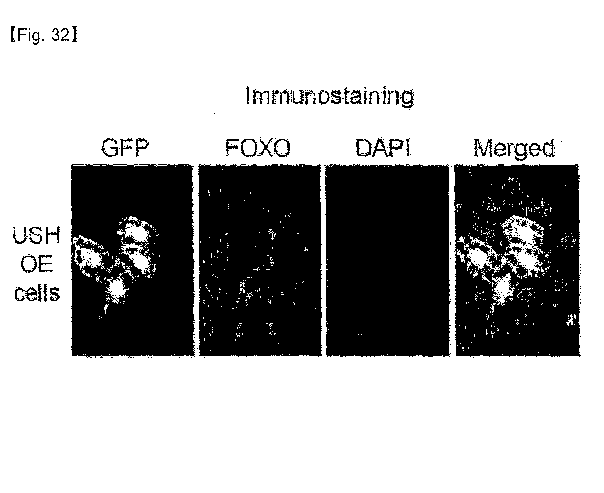

In order to determine whether USH negatively regulates insulin signaling, the inventors produced mosaic clones of fat cells overexpressing USH in the same manner as described in Example 13-1. The USH-overexpressing mosaic clones were transfected with tGPH vector in the same manner as described in Example 12, and then were subjected to immuno-staining using anti-GFP antibody (membrane staining, PI3K activity measurement) and anti-LacZ antibody (USH expressing cell staining). As a result, USH-overexpressing cells were smaller in size and showed significantly lower tGPH signals in the membrane (see FIG. 30 and 31), and higher FOXO signals in the nucleus than did the neighboring wild-type cells (see FIG. 32).

### <14-2> Determination of USH regulation location of in insulin signaling pathway

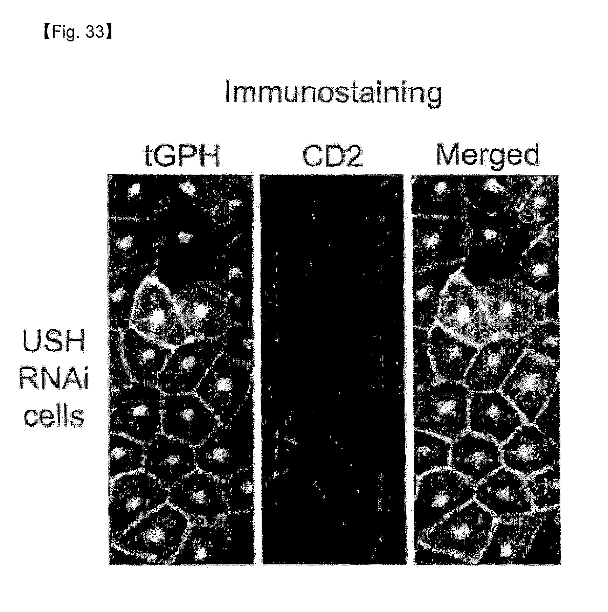

The inventors produced mosaic fat cells expressing dsRNA against ush to observe the knockdown phenotype of ush in the same manner as described in Example 13-1. The mosaic ush mutant cells were transfected with tGPH vector in the same manner as described in Example 12, and then were subjected to immuno-staining using anti-GFP antibody (membrane staining, PI3K activity measurement) and anti-CD2 antibody (miR-null cell staining). As a result, the tGPH signal was significantly enhanced (see FIG. 33), and the nuclear FOXO signals decreased (see FIG. 34). From this result, it can be found that USH inhibits insulin signaling upstream of or in parallel with PI3K in a cell-autonomous manner.

### Example 15. Determination of insulin signaling suppression in fat body of miR-8 null larvae by ush expression increase

Excessive insulin signaling is known to reduce the levels of insulin receptor (Inr) and cytohesin Steppke (step) through negative feedback by FOXO (Fuss et al., 2006; Puig and Tjian, 2005). The inventors examined whether reduced insulin signaling caused by the absence of miR-8 could be rescued by knockdown of USH. From fat body cells of CgG4, mir8 CgG4, mir8 CgG4>mir8 and mir8 Cg>ush i flies, total RNA was separated, and quantitative RT-PCR on FOXO target genes Inr and step mRNA was performed in the same manner as described in Example 4. The primers used in this process were noted in Table 7. As a result, it was found that these two targets of FOXO were upregulated in the fat body of miR-8 null larvae, whereas reintroduction of miR-8 dramatically reduced their expression (see FIG. 35). Especially, the mRNA levels of the FOXO target genes Inr and step in mir8 Cg>ush i fat bodies were restored (see FIG. 35). Thus, it can be determined that the defect of insulin signaling in the fat body of miR-8 null larvae is at least partially attributable to elevated ush levels.

**[Table 7]**

| gene | Primer | | | |
|---|---|---|---|---|
| | sense primer | | antisense primer | |
| Inr | SEQ ID: 106 | 5'-AACAGTGGCGGATTCGGTT-3' | SEQ ID:107 | 5'-TACTCGGAGCATTGGAGGCAT-3' |
| step | SEQ ID:108 | 5'-CACCAGGAACTTCTTGAATC-3' | SEQ ID:109 | 5'-TTCTTTGTACCAGGATGTCA-3' |

### Example 16. Regulation of PI3K in human cell lines by FOG2, a target gene of miR-200

### <16-1> Determination of regulation on mutation in mutant miR target site in fog2 3'UTR, by miR-200

From the detection result of miRNA target genes in Example 7, it was found that the ush mRNA has one predicted binding site for miR-8, whereas the mammalian ortholog of ush, FOG2, has at least three predicted binding sites for miR-200 family miRNAs (see FIG. 36 and 37). The inventors examined whether human miR-200 family miRNAs regulate the expression by directly binding to 3' UTR of fog2 mRNA in the same manner as drosophila miR-8 miRNA regulates the expression by directly binding to 3' UTR of ush mRNA. Specifically, in order to obtain cDNA of FOG2, represented by SEQ ID:110, from K562 cell lines, total RNA was extracted using RNeasy mini kit (QIAGEN, US). Then, 1 µg of total RNA and 1 µℓ of Oligo-dT (Invitrogen, US) were charged with up to 50 µℓ of distilled water, and placed into SuperScript First-Strand Synthesis System (Invitrogen, US), followed by RT-PCR reaction. Through the reaction at 65□ for 6 min, at 40 for 5 min, at 42□ for 60 min, at 95□ for 5 min, and at 4□ for 5 min, cDNA was synthesized. By using the cDNA (3~5 µg) as a template, and using a primer pair of SEQ ID:111 (5'-GGATCCATGTCCCGGCGAAAGCAAAGC-3') and SEQ ID:112(5'-GCGGCCGCTCATTTGACATGTTCTGCTGGATG -3') and PCR Mastermix (Promega, US), PCR was performed. After the reaction was completed, the PCR product of FOG2 was cleaved by BamHI and Notl restriction enzymes, respectively, and was cloned into pCK-flag vector (Heo I et al., Cell, 2009) cleaved by the same restriction enzymes so as to produce "pCK-FOG2" vector. By using pCK-FOG2, primers noted in Table 8, and QuickChange Site-Directed Mutagenesis kit, one (FOG2 m1, FOG2 m2, FOG2 m3), two ((FOG2 m12, FOG2 m23) or three (FOG2 m123) mutants were introduced into three miR-200 target sites of FOG2 3'UTR. Hela cell lines were transfected with wild-type FOG2(FOG2 wt), FOG2 m1, FOG2 m2, FOG2 m3, FOG2 m12, FOG2 m23 or FOG2 m123, together with renilla luciferase expression vector and synthesized siGFP, miR-141/200a or miR-200b/c/429. Then, the activity of luciferase was measured in the same manner as described in Example 8-2. The synthesized miRNA include a mixture of the same levels of miRNAs including respective seed sequences. As a result, when all the putative target sites in 3'UTR of fog2 mRNA were mutated (see FIG. 38, m123), expression of luciferase was not inhibited by miR-200 family miRNAs. This indicates that the predicted sites in the 3'UTR of fog2 mRNA are, therefore, responsible for the regulation of miR-200-mediated FOG2 expression inhibition. Meanwhile, miR-200a and miR-141 that have one nucleotide mismatch in the seed sequence suppressed the expression of luciferase, albeit less effectively than the other members did. This suggests that the noncanonical target sites may also cause a reduction of target gene expression (Bartel, DP 2009, Cell 136, 215-233; Brennecke, J et al., 2005, PLoS Biol 3, e85).

### <16-2> Determination of correlation between the expression of FOG2 protein and miR-200c cluster miRNAs in different tissues

FOG2 is expressed in the heart, brain, testes, liver, lung, and skeletal muscle (Holmes et al., 1999; Lu et al., 1999; Svensson et al., 1999; Tevosian et al., 1999). Despite its relatively broad expression in adult tissues, little is known about the function of FOG2 beyond its role in embryonic heart development (Fossett and Schulz, 2001). Meanwhile, the miR-200 miRNAs have also been reported to be expressed in various adult organs, including pituitary gland, thyroid, pancreatic islet, testes, prostate, ovary, breast, and liver (Landgraf et al., 2007). The inventors confirmed a correlation between the expression of FOG2 protein and miR-200c cluster miRNAs in human cell lines derived from different organs. For this, they performed western blotting and Northen blotting so as to determine the expression of FOG2 protein and miR-200 family miRNAs in cell lines, respectively. Specifically, in HepG2, Hep3B, Huh7, FAO, AsPC1, PANC1, SW480, HCT116, HCT116p53KD, MCF7, MDAMB231, U2OS and Hela cell lines, western blotting was performed in the same manner as described in Example 4, by using anti-FOG2 antibody (Santa cruz) and anti-GAPDH antibody (Santa cruz). Also, from HepG2, Hep3B, Huh7, FAO, AsPC1, PANC1, SW480, HCT116, HCT116p53KD, MCF7, MDAMB231, U2OS and Hela cell lines, total RNA was extracted by using Trizol reagent (Invitrogen, US), and Northen blotting was performed in the same manner as described in Example 5. Herein, oligonucleotides corresponding to miR-141 and miR-200c were end-labeled with [γ32-ATP] and used as probes for Northen blotting. As a result, there is generally a negative correlation between miR-200c cluster members and FOG2 in expression level, consistent with a suppressive role for miR-200 in FOG2 regulation (see FIG. 39).

**[Table 8]**

| gene | primer | | | |
|---|---|---|---|---|
| | sense primer | | antisense primer | |
| m1 | SEQ ID:113 | 5'-TTAATTTATTTTACC AGTGACATTCATAGCT GTGGTT-3' | SEQ ID:114 | 5'-AACCACAGCTAT GAATGTCACTGGTA AAATAAATTAA-3' |
| m2 | SEQ ID:115 | 5'-ACAAAATTACTGGA AGTGACATTGTAAGTA ATGAGG-3' | SEQ ID:116 | 5'-CCTCATTACTTAC AATGTCACTTCCAG TAATTTTGT-3' |
| m3 | SEQ ID:117 | 5'-TTGTAAGTAATGAG GTGACGTTAATCAGTT TTATCT-3' | SEQ ID:118 | 5'-AGATAAAACTGAT TAACGTCACCTCATT ACTTACAA-3' |

### Example 17. Determination of specific regulation of P13K-Akt-FOXO signaling by miR-200

### <17-1> Determination of connection of FOG2 to an insulin signaling process

The inventors examined whether FOG2 is in actuality reduced by miR-200 miRNAs, and whether human miR-200 miRNAs have a conserved role in the modulation of insulin signaling, as in the case of fly miR-8 miRNA. Hepatocellular carcinoma Huh7 cells that express relatively low but detectable levels of miR-200 and FOG2 (see FIG. 39) were transfected with 30 nM of synthesized siGFP, miR-141/200a or miR-200b/c/429, and then, western blotting was performed by using anti-FOG2 antibody, anti-p-Akt antibody, anti-Akt antibody and anti-GAPDH antibody in the same manner as described in Example 4. As a result, the FOG2 protein level was significantly reduced (see FIG. 40). In addition, pancreatic cancer AsPC1 cells that express relatively high levels of miR-200 miRNAs were treated with 200 nM of 2'-O-methyl oligonuclelotides antisense (table 9, samchonri) against siGFP, miR-141/200a or miR-200b/c/429, and then western blotting was performed in the same manner as described above. As a result, the FOG2 protein level was increased (see FIGs. 41 and 39). These data demonstrate that FOG2 is an authentic target of endogenous miR-200 miRNAs. Transfection of miR-200 family increased phosphorylation of Akt as compared to the case of transduction of a negative control (see FIG. 40). Meanwhile, transfection of miR-200 family inhibitors reduced Akt phosphorylation (see FIG. 41). Further, FAO cell lines were treated with siGFP or siFOG2 (SEQ ID:119: 5'-UUAUUCAAGUCCAUCUUCCdTdT3', Samchonri), and western blotting was performed in the same manner as described above. As a result, knockdown of FOG2 increased p-Akt levels, while mimicking the effect of miR-200 family miRNAs (see FIG. 42).

**[table 9]**

| - | SEQ ID: | Base sequence |
|---|---|---|
| anti-siGFP | SEQ ID:120 | 5'-AACAUCGCCAUCUAAUUCA-3' |
| anti-miR-141 | SEQ ID:121 | 5'-CCAUCUUUACCAGACAGUGU UA-3' |
| anti-miR-200a | SEQ ID:122 | 5'-ACAUCGUUACCAGACAGUGU UA-3' |
| anti-miR-200b | SEQ ID:123 | 5'-UCAUCAUUACCAGGCAGUAU UA-3' |
| anti-mi R-200c | SEQ ID:124 | 5'-UCCAUCAUUACCCGGCAGUAUUA-3' |
| anti-miR-429 | SEQ ID:125 | 5'-ACGGUUUUACCAGACAGUAUUA-3' |

### <17-2> Determination of a role of FOG2 in an insulin signaling process

The inventors transfected Hep3B cell lines with pCK-flag (empty vector) and pCK-FOG2 produced in Example 16-1, and tested the effect of FOG2 on PI3K activity by immunocomplex kinase assay using an antibody against p85α (see FIG. 43). Specifically, Hep3B cell lines were transfected with pCK-flag (empty vector) and pCK-FOG2 produced in Example 16-1, and the medium was replaced with serum-free medium or medium containing 100 ng/Mℓ of IGF-1. After 24 hr, the cells were treated with lysis buffer (137 mM NaCl, 20 mM Tris-HCI(pH 7.4), 1 mM CaCl2, 1 mM MgCl2, 0.1 mM sodium orthovanadate, and 1 % NP-40), and the protein was separated. Then, the test sample was treated with anti-p85α monoclonal antibody (Santa Cruz) so as to immunoprecipitate PI3K. The precipitated antibody-Pl3K immunocomplexes were mixed with protein A-Sepharose beads (Pharmacia, Sweden), washed twice with lysis buffer, and washed twice with (0.1 M Tris-HCI(pH 7.4), 5 mM L.iCI, and 0.1 mM sodium orthovanadate). PI3K activity was assayed by adding protein-A sepharose beads/antibody-PI3K immunocomplexes, 10 µg of sonicated PIP2 (Calbiochem, US) and 1 µℓ of [γ-32P]ATP(500 µCi/ml) in 60 µℓ of kinase assay buffer [10 mM Tris-HCI(pH 7.4), 150 mM NaCl, 1 mM sodium orthovanadate and 10 µℓ of 100 mM MgCl2]. The reactions were terminated after 20 min at 37□ by the addition of 20 µℓ of 6N HCI. The lipids were extracted with CHCI3:MeOH (1:1) and were analyzed using scintillation counter. For in vitro PI3K activity assay, the immunocomplexes were incubated with purified His-tagged FOG2 proteins for 2 hr at 4□.

As a result, when Hep3B cells were transduced with a FOG2-expression plasmid, IGF-1 (insulin like growth factor-1) failed to induce PI3K activity, indicating that POG2 suppresses PI3K (see FIG. 43).

Also, in cell lines which were transfected and treated or not treated with IGF-1 in the same condition as described above, western blotting was performed by using anti-p-Akt antibody, anti-Akt antibody and anti-GAPDH antibody in the same manner as described Example 4. As a result, in IGF-1-treated cells, treatment of FOG2 reduced phosphorylation of Akt as compared to non-treatment of FOG2 (see FIG. 44).

### <17-3> Determination of influence on insulin signaling pathway by miR-8

The inventors analyzed the effect of miR-200 miRNAs on the downstream transducers of Akt (see FIGs. 44 to 47). Because Akt represses FOXO activity, the inventors used a luciferase reporter plasmid (pFK1tk-luc) (Biggs, WH et al., 1999, Proc Natl Acad Sci U S A 96, 7421-7426) containing eight FOXO-binding sites to determine the level of FOXO activity in cultured cells. Specifically, Hep3B cells were transfected with synthesized siGFP, miR-141, miR-200a, miR-200b, miR-200c, miR-429 or siFOG2 together with pFK1tk-luc vector, and then luciferase activity was measured in the same manner as described Example 8-2. Also, Hep3B cells were transfected with luciferase, or oligonucleotides having complementary sequence to miR-141, miR-200a, miR-200b, miR-200c or miR-429 together with pFK1tk-luc vector. Then, the luciferase activity was measured in the same manner as described above. Further, Hep3B cell lines were transfected with pCK-flag (empty vector) and pCK-FOG2 produced in Example 16-1, and the luciferase activity was measured in the same manner as described above. The activity of luciferase in Hep3B cells was significantly repressed by transduction of miR-200 miRNAs and by FOG2 knockdown (see FIG. 45). Further, treating the cells with miR-200 inhibitors elevated FOXO activity (see FIG. 46). Consistently, when FOG2 was overexpressed, FOXO activity was upregulated (see FIG. 47).

### <17-4> Determination of influence on insulin signaling pathway, by FOG2 expression regulation by miR-8

The inventors examined whether miR-200 has a significant influence on insulin signaling pathway. HepG2 cell lines were transfected with pCK-flag (empty vector) or pCK-FOG2 produced in Example 16-1, and the medium was replaced with serum-free medium or medium containing 100 ng/Mℓ of IGF-1. Then, on the cells, western blotting was performed in the same manner as described in Example 4 by using anti-p-ERK antibody (Sigma, US), anti-ERK antibody (Sigma, US) and anti-GAPDH antibody. As a result, in contrast to PI3K signaling pathway components, the level of phosphorylated Erk was not significantly impaired, irrespective of treatment of FOG2 (see FIG. 44). Also, Huh7 cell lines were transfected with synthesized siGFP, miR-141/200a or miR-200b/c/429, and not treated with anything, or treated with 10 µM of Ly294002 (PI3K inhibitor), SB600125 (JNK inhibitor) or U0126 (Mek1 inhibitor) each. Then, FOXO activity was measured in the same manner as described in Example 17-3. As a result, inhibitors of JNK or Meek1/2 did not affect miR-200-mediated FOXO regulation. On the other hand, when the cells were treated with PI3K inhibitor, miR-200 did not reduce FOXO activity (see FIG. 45). From this result, it was found that miR-200 specifically regulates PI3K-Akt-FOXO signaling.

### Example 18. FOG2 functioning as negative regulator of P13K

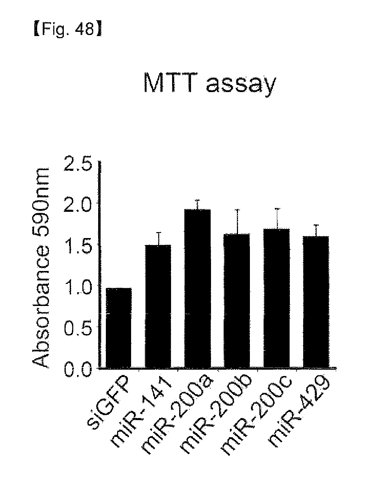

Because stimulation of PI3K and Akt is known to facilitate cell proliferation and antagonize apoptosis (Pollak, 2008), the inventors measured cellular viability with the MTT (3-(4,5-dimethylthiazol-2-yl)-2,5-diphenyltetrazolium bromide) assay in Hep3B cells. Specifically, Hep3B cells were plated in a 96-well plate, and transfected with 30 nM of synthesized siGFP, miR-141, miR-200a, miR-200b, miR-200c, mi-429 or siFOG2. Then, to the plate, 50 µl of MTT solution (2 mg/ml)(Promega, US) was added per each well, followed by culturing at 37□ for 1 hour. Then, each well was added with 150 µl of DMSO, and measured by a microplate reader (Bio-rad, US) at a wavelength of 590 nm. Also, Hep3B cells were transfected with 200 nM of luciferase, or 200 nM of oligonucleotides having complementary sequences to miR-141, miR-200a, miR-200b, miR-200c or miR-429, and then were subjected to MTT assay in the same manner as described above. As a result, introduction of miR-200 miRNAs increased cell viability (see FIG. 48), whereas introduction of miRNA inhibitors produced the opposite effect (see FIG. 49).

In order to investigate the action mechanism of FOG2, the inventors performed western blotting against p85α, p110 and IRS-1 following p85α immunoprecipitation in Hep3B cell lines (see FIG. 50). Especially, when FOG2 was expressed, reduced amounts of p110 and IRS-1 were coprecipitated with p85α. Thus, it was found that FOG2 acts as a negative regulator of PI3K by interfering with the formation of an IRS-1/p85αp110 complex.

### Example 19. FOG2 inhibiting PI3K by directly binding to p85α

### <19-1> Determination of interaction FOG2 and PI3K within cells

Although FOG2 is thought to be a nuclear transcriptional coregulator, several studies have reported that FOG2 also localizes to the cytoplasm (Bielinska et al., 2005; Clugston et al., 2008). To confirm this finding, the inventors separated nucleus and cytoplasm fractions from Hela or PANC1 cells, and separated proteins from each fraction. Then, western blotting was performed by using anti-FOG2 antibody, anti-Lamin antibody (nucleus fraction)(Santa Cruz, US) and anti-Tubulin antibody (cytoplasm fraction)(Santa Cruz) in the same manner as described in Example 4. As a result, FOG2 was observed predominantly in the cytoplasm rather than in the nucleus (see FIG. 51). When HepG2 cells were subjected to immuno-staining using DAPI staining, anti-FOG2 antibody and anti-p85α antibody in the same manner as described in Example 5, cytoplasmic localization of FOG2 was shown (see FIG. 52). This suggests a cytoplasmic role of FOG2.

Given that FOG2 suppresses PI3K and colocalizes with p85α (see FIGs. 38 to 54), the inventors suspected that FOG2 may interact with PI3K. When PANC1 cells were treated or not treated with 1 µg of (Santa cruz), immunoprecipitation was performed in the same manner as described in 17-2. Then, by using anti-FOG2 antibody and anti-p85α antibody, western blotting was performed in the same manner as described in Example 4. When endogenous expression of FOG2 was examined, a significant amount of p85α, the regulatory subunit of PI3K, was coprecipitated with anti-FOG2 antibody (see FIG. 56).

### <19-2> Determination of in vitro interaction between FOG2 and PI3K

Hep3B cell lines were transfected with pCK-flag (empty vector) or pCK-FOG2 produced in Example 10-1, and the medium was replaced with serum-free medium or medium containing 100 ng/Mℓ of IGF-1. Then, on the cells, immunoprecipitation was performed by using anti-85 antibody in the same manner as described in Example 17-2, and western blotting was performed by using anti-p85α antibody, anti-p110 antibody and anti-FOG2 antibody in the same manner as described in Example 4. As a result, interaction between FOG2 and p85α was also observed even when the expression of two proteins was induced in FLAG-tagged form (see FIGs. 54 and 55).

### Example 20. Function of miR-8/miR-200 and USH/FOG2 conserved in both species

### <20-1> mapping of a domain of FOG2 binding to p85α

In order to map the domain of FOG2 binding to p85α, the inventors, by using cDNA of FOG2 (from Example 16-1), as a template, and the following primers of respective truncated mutants of FOG, polynucleotides on respective FOG truncated mutants were amplified:
FOG2[1-412](SEQ ID:126): sense primer; SEQ ID:127, 5'-GGATCCATGTCCCGGCGAAAGCAAAGC-3', antisense primer; SEQ ID:128, 5'-GCGGCCGCGTGGCTGGCTGTAAGCTGTC-3',
FOG2[413-789](SEQ ID:129) sense primer; SEQ ID:130, 5'-GGATCCCAGACTTATTGACCAGAAG-3', antisense primer; SEQ ID:131 5'-GCGGCCGCGATATCACATCTTGGGTGGTAG-3',
FOG2[802-1151](SEQ ID:132) sense primer; SEQ ID:133, 5'-GGATCCCTCTGACGATCAACAAGTG-3', antisense primer; SEQ ID-134 5'-GCGGCCGCTCATTTGACATGTTCTGCTGCATG-3',
FOG2[1-506](SEQ ID:135) sense primer; SEQ ID:136, 5'-GGATCCATGTCCCGGCGAAAGCAAAGC-3', antisense primer; SEQ ID:137, 5'-GCGGCCGCTCATTTGACATGTTCTGCTGCATG-3', and
FOG2[1-789](SEQ ID:138) sense primer; SEQ ID:139, 5'-GGATCCATGTCCCGGCGAAAGCAAAGC-3', antisense primer; SEQ ID:140, 5'-GCGGCCGCTCATTTGACATGTTCTGCTGCATG-3'.

The above obtained PCR product was cleaved by BamHI and Notl restriction enzymes, and cloned into pCK-flag vector cleaved by the same restriction enzymes so as to produce expression vector for FOG mutants of "pCK-FOG2_1-412", "pCK-FOG2_413-789", "pCK-FOG2_802-1151", "pCK-FOG2_1-506" and "pCK-FOG2_1-789",

The obtained mutants containing a FLAG-tag in the N terminal were expressed respectively in HepG2, and immunoprecipitation was performed using anti-FLAG antibody in the same manner as described in Example 17-2. Then, anti-p85α antibody and anti-FOG2 antibody were used to perform analysis in the same manner as described in Example 4. As a result, it was confirmed that the middle region of FOG2 (507-789 aa) mediates the interaction with p85α (see FIG. 55).

### <20-2> Determination of PI3K activity suppression of p85α binding-FOG2 domain

The inventors examined whether the middle region is sufficient to inhibit PI3K when the above produced mutant FOG2 proteins were expressed in HepG2 cells (see FIG. 56). As a result, the middle region significantly suppressed PI3K whereas neither the N-terminal part nor the C-terminal part had a significant effect on PI3K activity (see FIG. 56).

### <20-3> Direct binding of FOG2 to p85α conserved across the phyla

in order to test whether FOG2 directly binds to p85α, the inventors cleaved PCR product on p85α gene (obtained from Example 20-1) by BamHI and EcoRI, and cloned into pGEX5X-1 vector (GE Healthcare, US) cleaved by the same restriction enzymes so as to produce GST-fused recombinant p85α expression vector. The expression vector was transfected into BL21 (Promega, US) through electroporation, and according to a known method, the FOG2 protein was expressed and purified from bacteria (Studier, F.W. et al., 1986, J. Mol. Biol. 189, 113-130), and was used in the following in vitro binding assay, along with purified GST-fused recombinant p85α protein. The recombinant FOG2 protein contains the middle region of FOG2 (413-789 aa) specifically bound to recombinant p85α (see FIG. 57).

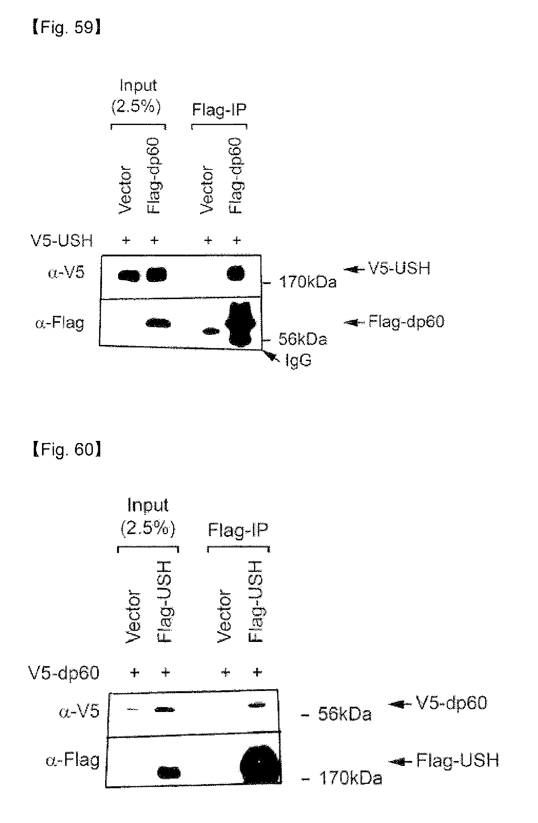

The inventors investigated whether FOG2 can directly inhibit p85α by performing an in vitro PI3K assay using recombinant FOG2. Addition of the recombinant FOG2 protein containing the middle region (tro PI3K assay) to the immunoprecipitated PI3K complex significantly inhibited the PI3K activity (see FIG. 58). This result suggests that direct binding of FOG2 to p83 leads to the inhibition of PI3K activity. Especially, the inventors found that drosophila USH physically interacted with drosophila p60 (dp60; the fly ortholog of p85α) when dp60 was co-expressed with USH in human HEK293T cells (see FIGs. 59 and 60). From this result, it can be found that the action mechanism of USH/FOG2 may be conserved across the phyla (see FIG. 61).

### [Industrial applicability]

It was determined that when in human cancer cell lines, expression of miR-200 was inhibited or FOG2 was expressed, PI3K activity promoting cell growth was reduced. Thus, miR-200 and FOG2 can be usefully utilized in screening of an insulin signaling regulator such as an anti-cancer agent.

## Claims

1. A method of screening an insulin signaling regulator, the method comprising the steps of:
1) treating cell lines expressing miR-200 family miRNAs or miR-8 miRNA with a testing compound;
2) measuring an expression level or an activity of FOG2 or USH protein in cells treated with the testing compound in the step 1); and
3) identifying the testing compound by which the expression or the activity of the FOG2 or USH protein in the cells in the step 1) has been changed as compared to a control.

2. The method as claimed in claim 1, wherein the cell lines expressing the miR-200 family miRNAs are human cell lines derived from tissues of heart, brain, testicle, liver, lung and skeletal muscle.

3. The method as claimed in claim 1, wherein the cell lines expressing the miR-8 miRNA are drosophila cell lines derived from fat body tissues.

4. The method as claimed in claim 1, wherein the testing compound is any one selected from the group including natural compounds, synthesized compounds, RNA, DNA, polypeptides, enzymes, proteins, ligands, antibodies, antigens, metabolites of bacteria or fungi, and bioactive molecules.

5. The method as claimed in claim 1, wherein in the step 2), the expression level of the FOG2 or USH protein is measured by any one method selected from the group including western blotting, immunostaining, fluorescent staining, and reporter assay.

6. The method as claimed in claim 1, wherein in the step 2), the activity of the FOG2 or USH protein is measured by any one method selected from the group including i) a method of measuring an activity of a p85a/p110/IRS-1 complex; ii) a method of measuring an expression level of FOXO mRNA or protein; and iii) a method of measuring cell growth and proliferation.

7. The method as claimed in claim 6, wherein the activity of the p85α/p110/IRS-1 complex is measured by measuring a phosphorylation level of AKT protein.

8. A method of screening an insulin signaling regulator, the method comprising the steps of:
1) treating cell lines expressing FOG2 or USH with a testing compound;
2) measuring an expression level or an activity of FOG2 or USH protein in cells in the step 1); and
3) identifying the testing compound by which the expression or the activity of the FOG2 or USH protein in the cells in the step 1) has been changed as compared to a control.

9. The method as claimed in claim 8, wherein the cell lines expressing the FOG2 are human cell lines derived from tissues of heart, brain, testicle, liver, lung and skeletal muscle.

10. The method as claimed in claim 8, wherein the cell lines expressing the USH are drosophila cell lines derived from fat body tissues.

11. The method as claimed in claim 8, wherein the testing compound is any one selected from the group including natural compounds, synthesized compounds, RNA, DNA, polypeptides, enzymes, proteins, ligands, antibodies, antigens, metabolites of bacteria or fungi, and bioactive molecules.

12. The method as claimed in claim 8, wherein in the step 2), the expression level of the FOG2 or USH protein is measured by any one method selected from the group including western blotting, immunostaining, fluorescent staining, and reporter assay.

13. The method as claimed in claim 8, wherein in the step 2), the activity of the FOG2 or USH protein is measured by any one method selected from the group including i) a method of measuring an activity of a p85α/p110/IRS-1 complex; ii) a method of measuring an expression level of FOXO mRNA or protein; and iii) a method of measuring cell growth and proliferation.

14. The method as claimed in claim 13, wherein the activity of the p85α/p110/IRS-1 complex is measured by measuring a phosphorylation level of AKT protein.

15. A method of screening an insulin signaling regulator, the method comprising the steps of:
1) bringing FOG2 protein into contact with p85α protein in the presence of a testing compound;
2) measuring a degree of binding of the FOG2 protein to the p85α protein; and
3) identifying the testing compound by which the degree of binding of the FOG2 protein to the p85α protein in vitro in the step 2) has been changed as compared to a control not treated with the testing compound.

16. A method of screening an insulin signaling regulator, the method comprising the steps of:
1) bringing USH protein into contact with dp60 protein in the presence of a testing compound;
2) measuring a degree of binding of the USH protein to the dp60 protein; and
3) identifying the testing compound by which the degree of binding of the USH protein to the dp60 (Drosophila p60) protein in vitro in the step 2) has been changed as compared to a control not treated with the testing compound.

17. The method as claimed in claim 15 or 16, wherein the testing compound is any one selected from the group including natural compounds, synthesized compounds, RNA, DNA, polypeptides, enzymes, proteins, ligands, antibodies, antigens, metabolites of bacteria or fungi, and bioactive molecules.

18. The method as claimed in claim 15 or 16, wherein in the step 3), the degree of binding between the proteins is measured by any one method selected from the group including SPR, protein chip, gel shift assay, immunoprecipitation assay, co-immunoassay, fluorescence immuno assay and radioimmuno assay.

19. A method of screening an insulin signaling regulator, the method comprising the steps of
1) treating cell lines expressing FOG2 protein with a testing compound;
2) measuring a degree of binding of FOG2 protein to p85α protein in cells treated with the testing compound in the step 1); and
3) identifying the testing compound by which the degree of binding of the FOG2 protein to the p85α protein in the cells in the step 1) has been changed as compared to a control not treated with the testing compound.

20. The method as claimed in claim 19, wherein the cell lines expressing the FOG2 are human cell lines derived from tissues of heart, brain, testicle, liver, lung and skeletal muscle.

21. The method as claimed in claim 19, wherein in the step 2), the degree of binding the FOG2 protein to the p85α protein is measured by any one method selected from the group including SPR, protein chip, gel shift assay, immunoprecipitation assay, co-immunoprecipitation assay, fluorescence immuno assay and radioimmuno assay.

22. A method of screening an insulin signaling regulator, the method comprising the steps of
1) treating cell lines expressing USH protein with a testing compound;
2) measuring a degree of binding of USH protein to dp60 protein in cells treated with the testing compound in the step 1); and
3) identifying the testing compound by which the degree of binding of the USH protein to the dp60 protein in the cells in the step 1) has been changed as compared to a control not treated with the testing compound.

23. The method as claimed in claim 22, wherein the cell lines expressing the USH are drosophila cell lines derived from fat body tissues.

24. The method as claimed in claim 22, wherein in the step 2), the degree of binding the USH protein to the dp60 protein is measured by any one method selected from the group including SPR, protein chip, gel shift assay, immunoprecipitation assay, co-immunoprecipitation assay, fluorescence immuno assay and radioimmuno assay.

25. The method as claimed in claim 19 or 22, wherein the testing compound is any one selected from the group including natural compounds, synthesized compounds, RNA, DNA, polypeptides, enzymes, proteins, ligands, antibodies, antigens, metabolites of bacteria or fungi, and bioactive molecules.

26. An insulin signaling regulating composition comprising a material changing an expression or an activity of FOG2 or USH protein, as an active ingredient.

27. The insulin signaling regulating composition as claimed in claim 26, comprising siRNA against FOG2, as the active ingredient.

28. The insulin signaling regulating composition as claimed in claim 26, wherein the siRNA against FOG2 is represented by sequence no 119.

29. A use of a material changing an expression or an activity of FOG2 or USH protein, in preparation of an insulin signaling regulating composition.

30. The use as claimed in claim 29, wherein siRNA against FOG2 is used in the preparation of the insulin signaling regulating composition.

31. The use as claimed in claim 30, wherein the siRNA against FOG2 is represented by sequence no 119.
